# EUROPEAN PATENT SPECIFICATION

(11) **EP 3 516 074 B1**
(45) Date of publication and mention of the grant of the patent: **05.08.2020**
(21) Application number: 17777231.6
(22) Date of filing: 22.09.2017
(51) Int. Cl.: C12Q 1/68

(54) **MIRNAS AS NON-INVASIVE BIOMARKERS FOR BREAST CANCER**
MIRNAS ALS NICHTINVASIVE BIOMARKER FÜR BRUSTKREBS
MIARN EN TANT QUE BIOMARQUEURS NON INVASIFS POUR LE CANCER DU SEIN

(30) Priority: 22.09.2016 EP 16190084
(43) Date of publication of application: 31.07.2019
(73) Proprietor: Hummingbird Diagnostics GmbH, 69120 Heidelberg (DE)
(72) Inventor: KAHRAMAN, Mustafa, 66111 Saarbrücken (DE); LAUFER, Thomas, 68535 Edingen-Neckarhausen (DE); KELLER, Andreas, 66346 Püttlingen (DE); RÖSKE, Anne, 67346 Speyer (DE)
(74) Representative: Geling, Andrea
(86) International application number: PCT/EP2017/074051
(87) International publication number: WO 2018/055093

(56) References cited:
- WO-A1-2014/006160
- MICHAEL G. SCHRAUDER ET AL: "Circulating Micro-RNAs as Potential Blood-Based Markers for Early Stage Breast Cancer Detection", PLOS ONE, vol. 7, no. 1, 5 January 2012 (2012-01-05) , page e29770, XP055323305, DOI: 10.1371/journal.pone.0029770
- ELENI VAN SCHOONEVELD ET AL: "Expression profiling of cancerous and normal breast tissues identifies microRNAs that are differentially expressed in serum from patients with (metastatic) breast cancer and healthy volunteers", BREAST CANCER RESEARCH, CURRENT MEDICINE GROUP LTD, GB, vol. 14, no. 1, 21 February 2012 (2012-02-21), page R34, XP021125980, ISSN: 1465-5411, DOI: 10.1186/BCR3127

### SEQUENCE LISTING

<110> Hummingbird Diagnostics GmbH
<120> MIRNAS AS NON-INVASIVE BIOMARKERS FOR BREAST CANCER
<130> 505-60 PCT
<150> EP 16 190 084.0
   <151> 2016-09-22
<160> 25
<170> PatentIn version 3.5
<210> 1
   <211> 23
   <212> RNA
   <213> Homo sapiens
<400> 1
   caaagugcug uucgugcagg uag 23
<210> 2
   <211> 23
   <212> RNA
   <213> Homo sapiens
<400> 2
   cagugcaaua guauugucaa agc 23
<210> 3
   <211> 20
   <212> RNA
   <213> Homo sapiens
<400> 3
   uacaguauag augauguacu 20
<210> 4
   <211> 22
   <212> RNA
   <213> Homo sapiens
<400> 4
   ggauaucauc auauacugua ag 22
<210> 5
   <211> 22
   <212> RNA
   <213> Homo sapiens
<400> 5
   ucguaccgug aguaauaaug cg 22
<210> 6
   <211> 21
   <212> RNA
   <213> Homo sapiens
<400> 6
   uacaguacug ugauaacuga a 21
<210> 7
   <211> 22
   <212> RNA
   <213> Homo sapiens
<400> 7
   uagguuaucc guguugccuu cg 22
<210> 8
   <211> 21
   <212> RNA
   <213> Homo sapiens
<400> 8
   cauuauuacu uuugguacgc g 21
<210> 9
   <211> 22
   <212> RNA
   <213> Homo sapiens
<400> 9
   ugagguagua gauuguauag uu 22
<210> 10
   <211> 23
   <212> RNA
   <213> Homo sapiens
<400> 10
   agcagcauug uacagggcua uga 23
<210> 11
   <211> 22
   <212> RNA
   <213> Homo sapiens
<400> 11
   agagguagua gguugcauag uu 22
<210> 12
   <211> 23
   <212> RNA
   <213> Homo sapiens
<400> 12
   uaaagugcuu auagugcagg uag 23
<210> 13
   <211> 22
   <212> RNA
   <213> Homo sapiens
<400> 13
   cagugcaaug uuaaaagggc au 22
<210> 14
   <211> 23
   <212> RNA
   <213> Homo sapiens
<400> 14
   ugugcaaauc uaugcaaaac uga 23
<210> 15
   <211> 20
   <212> RNA
   <213> Homo sapiens
<400> 15
   gcggagagag aauggggagc 20
<210> 16
   <211> 20
   <212> RNA
   <213> Homo sapiens
<400> 16
   uaacggccgc gguacccuaa 20
<210> 17
   <211> 22
   <212> RNA
   <213> Homo sapiens
<400> 17
   ucgggccugg gguuggggga gc 22
<210> 18
   <211> 22
   <212> RNA
   <213> Homo sapiens
<400> 18
   ugagguagua aguuguauug uu 22
<210> 19
   <211> 23
   <212> RNA
   <213> Homo sapiens
<400> 19
   uagugcaaua uugcuuauag ggu 23
<210> 20
   <211> 23
   <212> RNA
   <213> Homo sapiens
<400> 20
   uaaggugcau cuagugcaga uag 23
<210> 21
   <211> 23
   <212> RNA
   <213> Homo sapiens
<400> 21
   uaaggugcau cuagugcagu uag 23
<210> 22
   <211> 22
   <212> RNA
   <213> Homo sapiens
<400> 22
   ugauauguuu gauauauuag gu 22
<210> 23
   <211> 23
   <212> RNA
   <213> Homo sapiens
<400> 23
   cagugcaaug auauugucaa agc 23
<210> 24
   <211> 23
   <212> RNA
   <213> Homo sapiens
<400> 24
   agcagcauug uacagggcua uca 23
<210> 25
   <211> 17
   <212> RNA
   <213> Homo sapiens
<400> 25
   gugggggaga ggcuguc 17

## Description

The present invention relates to a method for diagnosing triple-negative breast cancer (TNBC) in a patient. Further, the present invention relates to the use of at least one polynucleotide for detecting at least one miRNA for diagnosing triple-negative breast cancer (TNBC) in a blood cellular fraction from a patient. Furthermore, the present invention relates to the use of at least one miRNA isolated from a blood cellular fraction from a patient for diagnosing triple-negative breast cancer (TNBC). In addition, the present invention relates to the use of a kit for diagnosing triple-negative breast cancer (TNBC) in a patient.

### BACKGROUND OF THE INVENTION

Breast cancer (BC) is one of the leading causes of cancer death among women worldwide. Breast cancer occurs mainly in women, but men can get it too. A number of studies on breast cancer detection and treatment have been published in the last years. Whereas most studies focus on the improvement of prognosis by improving therapies, one of the most promising approaches is to detect breast cancer at an early stage. Mammography and ultrasound are currently the standard diagnostic tools which have been proven to be successful for the detection of early stage breast cancer. They possess a sufficient specificity. Specificity of screening mammography is over 90%, but sensitivity ranges between 40% and 95% and is strongly dependent on several factors like breast density and professional experience of the examiner. For example, in women with very dense breast tissue, sensitivity of screening mammograms can be as low as 40 to 50%. Magnetic resonance imaging (MRI) of the breast could improve cancer detection in these cases, but this imaging technique is expensive and not routinely used for breast cancer screening. Therefore, intensive research is currently carried out to identify new non-invasive breast cancer detection methods. These new detection methods should supplement or even replace breast imaging. They should further improve detection rates and breast cancer screening compliance.

Today, miRNAs (microRNAs) as biomarkers play a key role in early diagnosis of various diseases. MiRNAs are small non-coding RNAs that regulate gene expression at the posttranslational level by degrading or blocking translation of messenger RNA (mRNA) targets. They are important players when it comes to regulate cellular functions and in several diseases, including cancer.

WO 2014/006160 A1 relates, for example, to miRNA oncologic biomarkers of breast cancer. MICHAEL G. SCHRAUDER et al. (PLOS ONE, Vol. 7, No. 1, pages 1 to 15) disclose circulating miRNAs as blood-based markers for early stage breast cancer detection. ELENI VAN SCHOONEVELD et al. (BREAST CANCER RESEARCH, CURRENT MEDICINE GROUP LTD, GB, Vol. 14, No. 1, pages 1 to 16) refer to the identification of miRNAs that are differentially expressed in serum from patients with (metastatic) breast cancer and healthy volunteers by expression profiling of cancerous and normal breast tissues.

The present inventors analysed miRNA expression profiles of early stage breast cancer patients compared to healthy controls. They identified single miRNAs which predict breast cancer with a high specificity, sensitivity, and accuracy. The present inventors also pursued a multiple biomarker strategy by implementing sets of miRNA biomarkers for the diagnosis of breast cancer. This approach could further increase specificity, sensitivity, and accuracy and, thus, the predictive power.
The present inventors further analysed miRNA expression profiles of patients prior to therapeutic treatment, in particular chemotherapy, and after therapeutic treatment, in particular chemotherapy. They surprisingly identified miRNAs which allow to determine whether a patient will respond to a therapeutic treatment, in particular chemotherapy, or not.
In addition, the present inventors identified a specific sample type, namely a blood cellular fraction comprising erythrocytes, leukocytes, and thrombocytes, as a special source of miRNAs having a high diagnostic potential.

### SUMMARY OF THE INVENTION

In a first aspect, the present invention relates to a method for diagnosing triple-negative breast cancer (TNBC) in a patient comprising the step of:
determining the level of at least one miRNA representative for triple-negative breast cancer in a blood cellular fraction from the patient,
wherein the at least one miRNA is selected from the group consisting of SEQ ID NO: 1 to SEQ ID NO: 23, and a sequence having at least 80% sequence identity thereto.

In a second aspect, the present invention relates to the use of at least one polynucleotide for detecting at least one miRNA for diagnosing triple-negative breast cancer (TNBC) in a blood cellular fraction from a patient, wherein the at least one miRNA is selected from the group consisting of SEQ ID NO: 1 to SEQ ID NO: 23.

In a third aspect, the present invention relates to the use of at least one miRNA isolated from a blood cellular fraction from a patient for diagnosing triple-negative breast cancer (TNBC), wherein the at least one miRNA is selected from the group consisting of SEQ ID NO: 1 to SEQ ID NO: 23, and a sequence having at least 80% sequence identity thereto.

In a fourth aspect, the present invention relates to the use of a kit for diagnosing triple-negative breast cancer (TNBC) in a patient comprising:
(i) means for determining the level of at least one miRNA representative for triple-negative breast cancer in a blood cellular fraction from the patient, and
(ii) optionally a tube for blood sample storage,
wherein the at least one miRNA is selected from the group consisting of SEQ ID NO: 1 to SEQ ID NO: 23, and a sequence having at least 80% sequence identity thereto.

Further described herein, but not encompassed by the present invention, is a method for determining whether a patient will respond to a therapeutic treatment of breast cancer comprising the step of:
determining the level of at least one miRNA associated with breast cancer in a blood sample from the patient.
Preferably, the at least one miRNA is selected from the group consisting of SEQ ID NO: 22, SEQ ID NO: 25, and a sequence having at least 80% sequence identity thereto.

Further described herein, but not encompassed by the present invention, is the use of at least one polynucleotide for detecting at least one miRNA for determining whether a patient will respond to a therapeutic treatment of breast cancer in a blood sample from the patient. Preferably, the at least one miRNA is selected from the group consisting of SEQ ID NO: 22 and SEQ ID NO: 25.

Further described herein, but not encompassed by the present invention, is the use of at least one miRNA isolated from a blood sample from a patient for determining whether the patient will respond to a therapeutic treatment of breast cancer.
Preferably, the at least one miRNA is selected from the group consisting of SEQ ID NO: 22, SEQ ID NO: 25, and a sequence having at least 80% sequence identity thereto.

Further described herein, but not encompassed by the present invention, is a kit for determining whether a patient will respond to a therapeutic treatment of breast cancer comprising:
(i) means for determining the level of at least one miRNA associated with breast cancer in a blood sample from the patient, and
(ii) optionally a tube for blood sample storage.
Preferably, the at least one miRNA is selected from the group consisting of SEQ ID NO: 22, SEQ ID NO: 25, and a sequence having at least 80% sequence identity thereto.

This summary of the invention does not necessarily describe all features of the present invention. Other embodiments will become apparent from a review of the ensuing detailed description.

### DETAILED DESCRIPTION OF THE INVENTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

The term "comprise" or variations such as "comprises" or "comprising" according to the present invention means the inclusion of a stated integer or group of integers but not the exclusion of any other integer or group of integers. The term "consisting essentially of' according to the present invention means the inclusion of a stated integer or group of integers, while excluding modifications or other integers which would materially affect or alter the stated integer. The term "consisting of' or variations such as "consists of' according to the present invention means the inclusion of a stated integer or group of integers and the exclusion of any other integer or group of integers.

The terms "a" and "an" and "the" and similar reference used in the context of describing the invention (especially in the context of the claims) are to be construed to cover both the singular and the plural, unless otherwise indicated herein or clearly contradicted by context.

The terms "microRNA" or "miRNA", as used herein, refer to single-stranded RNA molecules of at least 10 nucleotides and of not more than 35 nucleotides covalently linked together. Preferably, the polynucleotides of the present invention are molecules of 10 to 35 nucleotides or 15 to 35 nucleotides in length, more preferably of 16 to 28 nucleotides or 17 to 27 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally labels and/or elongated sequences (e.g. biotin stretches). The miRNAs regulate gene expression and are encoded by genes from whose DNA they are transcribed but miRNAs are not translated into protein (i.e. miRNAs are non-coding RNAs). The genes encoding miRNAs are longer than the processed mature miRNA molecules. The miRNAs are first transcribed as primary transcripts or pri-miRNAs with a cap and poly-A tail and processed to short, 70 nucleotide stem-loop structures known as pre-miRNAs in the cell nucleus. This processing is performed in animals by a protein complex known as the Microprocessor complex consisting of the nuclease Drosha and the double-stranded RNA binding protein Pasha. These pre-miRNAs are then processed to mature miRNAs in the cytoplasm by interaction with the endonuclease Dicer, which also initiates the formation of the RNA-induced silencing complex (RISC). When Dicer cleaves the pre-miRNA stem-loop, two complementary short RNA molecules are formed, but only one is integrated into the RISC. This strand is known as the guide strand and is selected by the argonaute protein, the catalytically active RNase in the RISC, on the basis of the stability of the 5' end. The remaining strand, known as the miRNA*, anti-guide (anti-strand), or passenger strand, is degraded as a RISC substrate. Therefore, the miRNA*s are derived from the same hairpin structure like the "normal" miRNAs. So if the "normal" miRNA is then later called the "mature miRNA" or "guide strand", the miRNA* is the "anti-guide strand" or "passenger strand".

The terms "microRNA*" or "miRNA*", as used herein, refer to single-stranded RNA molecules of at least 10 nucleotides and of not more than 35 nucleotides covalently linked together. Preferably, the polynucleotides of the present invention are molecules of 10 to 35 nucleotides or 15 to 35 nucleotides in length, more preferably of 16 to 28 nucleotides or 18 to 23 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally labels and/or elongated sequences (e.g. biotin stretches). The "miRNA*s", also known as the "anti-guide strands" or "passenger strands", are mostly complementary to the "mature miRNAs" or "guide strands", but have usually single-stranded overhangs on each end. There are usually one or more mispairs and there are sometimes extra or missing bases causing single-stranded "bubbles". The miRNA*s are likely to act in a regulatory fashion as the miRNAs (see also above). In the context of the present invention, the terms "miRNA" and "miRNA*" are interchangeable used.

The term "miRBase", as used herein, refers to a well established repository of validated miRNAs. The miRBase (www.mirbase.org) is a searchable database of published miRNA sequences and annotation. Each entry in the miRBase Sequence database represents a predicted hairpin portion of a miRNA transcript (termed mir in the database), with information on the location and sequence of the mature miRNA sequence (termed miR). Both hairpin and mature sequences are available for searching and browsing, and entries can also be retrieved by name, keyword, references and annotation. All sequence and annotation data are also available for download. The sequences of the miRNAs for diagnosing breast cancer described herein are based on miRBase version v21.

The term "nucleotides", as used herein, refers to structural components, or building blocks, of DNA and RNA. Nucleotides consist of a base (one of four chemicals: adenine, thymine, guanine, and cytosine) plus a molecule of sugar and one of phosphoric acid. The term "nucleosides" refers to glycosylamine consisting of a nucleobase (often referred to simply base) bound to a ribose or deoxyribose sugar. Examples of nucleosides include cytidine, uridine, adenosine, guanosine, thymidine and inosine. Nucleosides can be phosphorylated by specific kinases in the cell on the sugar's primary alcohol group (-CH2-OH), producing nucleotides, which are the molecular building blocks of DNA and RNA.

The term "polynucleotide", as used herein, means a molecule of at least 10 nucleotides and of not more than 35 nucleotides covalently linked together. Preferably, the polynucleotides of the present invention are molecules of 10 to 35 nucleotides or 15 to 35 nucleotides in length, more preferably of 16 to 28 nucleotides or 17 to 27 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally spacer elements and/or elongation elements described below. The depiction of a single strand of a polynucleotide also defines the sequence of the complementary strand. Polynucleotides may be single stranded or double stranded, or may contain portions of both double stranded and single stranded sequences. The term "polynucleotide" means a polymer of deoxyribonucleotide or ribonucleotide bases and includes DNA and RNA molecules, both sense and anti-sense strands. In detail, the polynucleotide may be DNA, both cDNA and genomic DNA, RNA, cRNA or a hybrid, where the polynucleotide sequence may contain combinations of deoxyribonucleotide or ribonucleotide bases, and combinations of bases including uracil, adenine, thymine, cytosine, guanine, inosine, xanthine, hypoxanthine, isocytosine and isoguanine. Polynucleotides may be obtained by chemical synthesis methods or by recombinant methods.
In the context of the present invention, a polynucleotide as a single polynucleotide strand provides a probe (e.g. miRNA capture probe) that is capable of binding to, hybridizing with, or detecting a target of complementary sequence, such as a nucleotide sequence of a miRNA or miRNA*, through one or more types of chemical bonds, usually through complementary base pairing, usually through hydrogen bond formation. Polynucleotides in their function as probes may bind target sequences, such as nucleotide sequences of miRNAs or miRNAs*, lacking complete complementarity with the polynucleotide sequences depending upon the stringency of the hybridization condition. There may be any number of base pair mismatches which will interfere with hybridization between the target sequence, such as a nucleotide sequence of a miRNA or miRNA*, and the single stranded polynucleotide described herein. However, if the number of mutations is so great that no hybridization can occur under even the least stringent hybridization conditions, the sequences are no complementary sequences. The polynucleotide variants including polynucleotide fragments or polynucleotide mutants and the miRNA variants including miRNA fragments or miRNA mutants are further defined below. Described herein are polynucleotides in form of single polynucleotide strands as probes for binding to, hybridizing with or detecting complementary sequences of miRNAs (targets), which are selected from the group consisting of SEQ ID NO: 1 to SEQ ID NO: 25.
The polynucleotide, e.g. the polynucleotide used as a probe for detecting a miRNA or miRNA*, may be unlabeled, directly labeled, or indirectly labeled, such as with biotin to which a streptavidin complex may later bind. The polynucleotide, e.g. the polynucleotide used as a probe for detecting a miRNA or miRNA*, may also be modified, e.g. may comprise an elongation (EL) element. For use in a RAKE or MPEA assay, a polynucleotide with an elongation element may be used as a probe. The elongation element comprises a nucleotide sequence with 1 to 30 nucleotides chosen on the basis of showing low complementarity to potential target sequences, such as nucleotide sequences of miRNAs or miRNAs*, therefore resulting in not to low degree of cross-hybridization to a target mixture. Preferred is a homomeric sequence stretch Nₙ with n = 1 to 30, i.e. 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30, and N = A or C, or T or G. Particularly preferred is a homomeric sequence stretch Nₙ with n = 1 to 12, i.e. 1, 2, 3, 4, 5, 6, 7, 8, 9, 10, 11, or 12, and N = A or C, or T or G. The polynucleotide, e.g. the polynucleotide used as a probe for detecting a miRNA or miRNA*, may be present in form of a tandem, i.e. in form of a polynucleotide hybrid of two different or identical polynucleotides, both in the same orientation, i.e. 5' to 3' or 3' to 5', or in different orientation, i.e. 5' to 3' and 3' to 5'. Said polynucleotide hybrid/tandem may comprise a spacer element. For use in a tandem hybridization assay, the polynucleotide hybrid/tandem as a probe may comprise a spacer (SP) element. The spacer element represents a nucleotide sequence with n = 0 to 12, i.e. 0, 1, 2, 3, 4, 5, 6, 7, 8, 9, 10, 11, or 12, nucleotides chosen on the basis of showing low complementarity to potential target sequences, such as nucleotide sequences of miRNAs or anti-miRNAs, therefore resulting in not to low degree of cross-hybridization to a target mixture. It is preferred that n is 0, i.e. that there is no spacer between the two miRNA sequence stretches.

The term "label", as used herein, means a composition detectable by spectroscopic, photochemical, biochemical, immunochemical, chemical, or other physical means. For example, useful labels include 32P, fluorescent dyes, electron-dense reagents, enzymes (e.g., as commonly used in an ELISA), biotin, digoxigenin, or haptens and other entities which can be made detectable. A label may be incorporated into nucleic acids at any position, e.g. at the 3' or 5' end or internally. The polynucleotide for detecting a miRNA (polynucleotide probe) and/or the miRNA itself may be labeled.

The term "stringent hybridization conditions", as used herein, means conditions under which a first nucleic acid sequence (e.g. polynucleotide in its function as a probe for detecting a miRNA or miRNA*) will hybridize to a second nucleic acid sequence (e.g. target sequence such as nucleotide sequence of a miRNA or miRNA*), such as in a complex mixture of nucleic acids. Stringent conditions are sequence-dependent and will be different in different circumstances. Stringent conditions may be selected to be about 5 to 10°C lower than the thermal melting point (Tm) for the specific sequence at a defined ionic strength pH. The Tm may be the temperature (under defined ionic strength, pH, and nucleic acid concentration) at which 50% of the probes complementary to the target hybridize to the target sequence at equilibrium (as the target sequences are present in excess, at Tm, 50% of the probes are occupied at equilibrium). Stringent conditions may be those in which the salt concentration is less than about 1.0 M sodium ion, such as about 0.01 to 1.0 M sodium ion concentration (or other salts) at pH 7.0 to 8.3 and the temperature is at least about 20°C for short probes (e.g., about 10-35 nucleotides) and up to 60°C for long probes (e.g., greater than about 50 nucleotides). Stringent conditions may also be achieved with the addition of destabilizing agents such as formamide. For selective or specific hybridization, a positive signal may be at least 2 to 10 times background hybridization. Exemplary stringent hybridization conditions include the following: 50% formamide, 5x SSC, and 1% SDS, incubating at 42°C, or, 5x SSC, 1% SDS, incubating at 65°C, with wash in 0.2x SSC, and 0.1% SDS at 65°C; or 6x SSPE, 10 % formamide, 0.01 %,Tween 20, 0.1 x TE buffer, 0.5 mg/ml BSA, 0.1 mg/ml herring sperm DNA, incubating at 42°C with wash in 05x SSPE and 6x SSPE at 45°C.

The term "antisense", as used herein, refers to nucleotide sequences which are complementary to a specific DNA or RNA sequence. The term "antisense strand" is used in reference to a nucleic acid strand that is complementary to the "sense" strand.

Residues in two or more polynucleotide s are said to "correspond" to each other if the residues occupy an analogous position in the polynucleotide structures. It is well known in the art that analogous positions in two or more polynucleotides can be determined by aligning the polynucleotide sequences based on nucleic acid sequence or structural similarities. Such alignment tools are well known to the person skilled in the art and can be, for example, obtained on the World Wide Web, for example, ClustalW (see www.ebi.ac.uk/clustalw) or Align (see http://www.ebi.ac.uk/emboss/align/index.html) using standard settings, preferably for Align EMBOSS::needle, Matrix: Blosum62, Gap Open 10.0, Gap Extend 0.5.

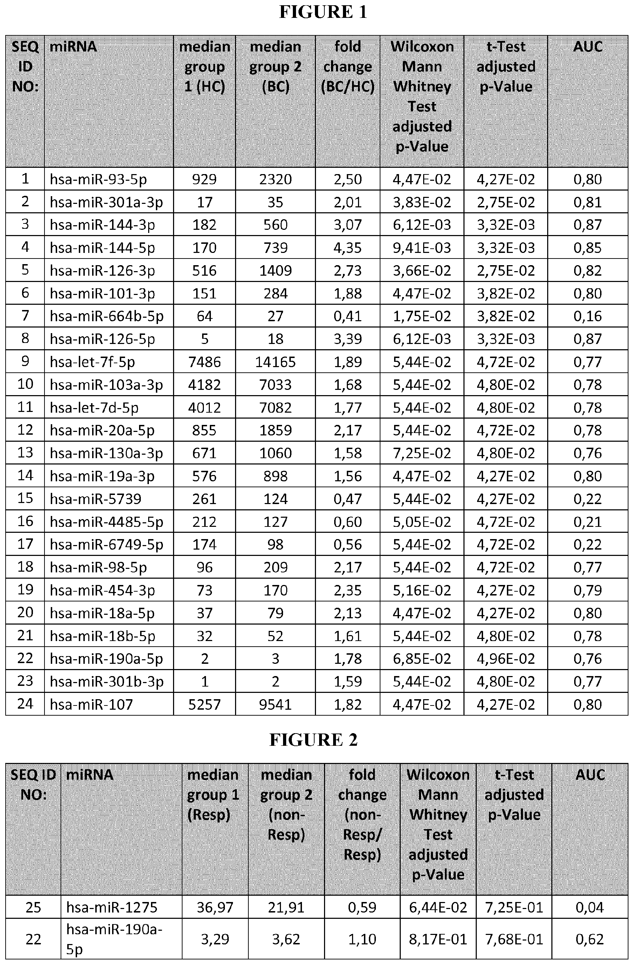

The term "at least one miRNA representative for breast cancer", as used herein, refers to a fixed defined miRNA which is known to be differentially regulated (or expressed) between subjects having breast cancer (diseased state) and healthy subjects and is, thus, representative for breast cancer. The term "at least one miRNA representative for breast cancer", as used herein, further refers to fixed defined miRNAs which are known to be differentially regulated between subjects having breast cancer (diseased state) and healthy subjects and are, thus, representative for breast cancer. As mentioned above, the present inventors analysed the level of miRNAs in blood samples of a cohort of controls (healthy subjects) and subjects having breast cancer. They succeeded in determining the miRNAs that are differentially regulated in blood samples from subjects having breast cancer compared to a cohort of controls (healthy subjects) (see experimental section for details). Said miRNAs are suitable biomarkers for the diagnosis of breast cancer in a patient (see, for example, Figure 1). Preferably, breast cancer is triple-negative breast cancer (TNBC).

The term "at least one miRNA associated with breast cancer", as used herein, refers to a fixed defined miRNA which is known to be differentially regulated between subjects known as responders to a therapeutic treatment of breast cancer and subjects known as non-responders to a therapeutic treatment of breast cancer and is, thus, associated with breast cancer. The term "at least one miRNA associated with breast cancer", as used herein, further refers to fixed defined miRNAs which are known to be differentially regulated between subjects known as responders to a therapeutic treatment of breast cancer and subjects known as non-responders to a therapeutic treatment of breast cancer and are, thus, associated with breast cancer. As already mentioned above, the present inventors analysed the level of miRNAs in blood samples of subjects having breast cancer before and after therapeutic treatment, in particular chemotherapy. They succeeded in determining the miRNAs that are differentially regulated in blood samples from subjects responding to a therapeutic treatment, in particular chemotherapy, or not (see experimental section for details). Said miRNAs are suitable biomarkers for determining whether a patient will respond to a therapeutic treatment of breast cancer (see, for example, Figure 2). Preferably, breast cancer is triple-negative breast cancer (TNBC).

The term "breast cancer", as used herein, refers to a cancer that develops from breast tissue. The term "breast cancer", as used herein, encompasses several different types of breast cancer which develop in different parts of the breast. In particular, the term "breast cancer", as used herein, includes non-invasive breast cancer, invasive breast cancer, and other types of breast cancer. Preferably, breast cancer is triple-negative breast cancer (TNBC).
The term "non-invasive breast cancer (also known as cancer or carcinoma *in situ*)", as used herein, refers to cancer that is found in the ducts of the breast and has not developed the ability to spread outside the breast. This form of cancer rarely shows as a lump in the breast that can be felt, and is usually found on a mammogram. The most common type of non-invasive cancer is ductal carcinoma in situ (DCIS).
The term "invasive breast cancer", as used herein, refers to a cancer type which has the ability to spread outside the breast, although this does not necessarily mean it has spread.
The most common form of breast cancer is invasive ductal breast cancer, which develops in the cells that line the breast ducts. Invasive ductal breast cancer amounts to about 80% of all breast cancer cases and is sometimes called "no special type".
The term "other types of breast cancer", as used herein, refers to other less common types of breast cancer including invasive lobular breast cancer, which develops in the cells that line the milk-producing lobules, inflammatory breast cancer, and Paget's disease of the breast.
It is possible for breast cancer to spread to other parts of the body, usually through the lymph nodes (small glands that filter bacteria from the body) or the bloodstream. If this happens, it is known as "secondary" or "metastatic" breast cancer.
The term "triple-negative breast cancer (TNBC)", as used herein, refers to any breast cancer, wherein breast cancer cells are tested negative for estrogen receptor (ER), progesterone receptor (PR), and/or Her2/neu. The term "triple-negative breast cancer (TNBC)", as used herein, further refers to any cancer that does not express the genes for estrogen receptor (ER), progesterone receptor (PR), and/or Her2/neu. This makes it more difficult to treat since most chemotherapies target one of the three receptors, so triple-negative cancer often requires combination therapies, e.g. drug and chemotherapy, or drug and radiation therapy.

The term "diagnosing breast cancer", as used herein, means determining whether a patient shows signs of or suffers from breast cancer. Preferably, breast cancer is triple-negative breast cancer (TNBC).

The term "patient", as used herein, refers to any subject for whom it is desired to know whether she or he suffers from breast cancer. In particular, the term "patient", as used herein, refers to a subject suspected to be affected by breast cancer. The patient may be diagnosed to be affected by breast cancer, i.e. diseased, or may be diagnosed to be not affected by breast cancer, i.e. healthy. The term "patient", as used herein, also refers to a subject which is affected by breast cancer, i.e. diseased. The patient may be retested for breast cancer and may be diagnosed to be still affected by breast cancer, i.e. diseased, or not affected by breast cancer anymore, i.e. healthy, for example after therapeutic intervention. It should be noted that a patient that is diagnosed as being healthy, i.e. not suffering from breast cancer, may possibly suffer from another disease not tested/known. The patient may be any mammal, e.g. a human. The human may be a female or male. Females are particularly preferred. Preferably, breast cancer is triple-negative breast cancer (TNBC).

The term "(control) subject", as used herein, refers to a subject known to be affected by breast cancer (positive control), i.e. diseased. The term "(control) subject", as used herein, also refers to a subject known to be not affected by breast cancer (negative control), i.e. healthy. Thus, the term "healthy subject", as used herein, means a subject which is known to be not affected by breast cancer. It should be noted that a (control) subject which is known to be healthy, i.e. not suffering from breast cancer, may possibly suffer from another disease not tested/known. The (control) subject may be any mammal, e.g. a human. The human may be a female or male. Females are particularly preferred. Preferably, breast cancer is triple-negative breast cancer (TNBC).

The term "treatment", in particular "therapeutic treatment", as used herein, refers to any therapy which improves the health status and/or prolongs (increases) the lifespan of a patient. Said therapy may eliminate the disease in a patient, arrest or slow the development of a disease in a patient, inhibit or slow the development of a disease in a patient, decrease the frequency or severity of symptoms in a patient, and/or decrease the recurrence in a patient who currently has or who previously has had a disease. The therapeutic treatment of breast cancer is selected from the group consisting of chemotherapy, surgery, and radiotherapy, preferably chemotherapy. Preferably, breast cancer is triple-negative breast cancer (TNBC).

The term "blood sample", as used herein, encompasses whole blood or a blood fraction such as serum, plasma, or blood cells. Preferably, the blood cells are erythrocytes, leukocytes and/or thrombocytes. The blood sample may have a volume of between 0.1 and 20 ml, more preferably of between 0.5 and 10 ml, i.e. 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, or 20 ml. Preferably, the blood sample is selected from the group consisting of whole blood and a blood cellular fraction.

The term "blood cellular fraction", as used herein, refers to a blood cellular portion which has been produced from whole blood by removing the extracellular fraction (serum and/or plasma). In other words, the blood cellular fraction is depleted of the extracellular blood components, such as serum and/or plasma. Preferably, the blood cellular fraction comprises/consists of erythrocytes, leukocytes, and thrombocytes.

The term "level", as used herein, refers to an amount (measured for example in grams, mole, or ion counts) or concentration (e.g. absolute or relative concentration) of the miRNA representative for breast cancer or associated with breast cancer described herein. The term "level", as used herein, also comprises scaled, normalized, or scaled and normalized amounts or values. Preferably, the level determined herein is the expression level.

The term "sensitivity", as used herein, refers to the number of true positive patients (%) with regard to the number of all patients (100%). The patients may be individuals having breast cancer. The sensitivity is calculated by the following formula: Sensitivity= TP/(TP+FN) (TP= true positives; FN=false negatives).

The term "specificity", as used herein, relates to the number of true negative individuals (%) with regard to the number of all healthy subjects (100%). The specificity is calculated by the following formula: Specificity= TN/(TN+FP) (TN= true negatives; FP=false positives).

The term "accuracy", as used herein, means a statistical measure for the correctness of classification or identification of sample types. The accuracy is the proportion of true results (both true positives and true negatives).

The result of each analysis group is usually calculated from a plurality of isolated samples, i.e. from at least 2 isolated samples, preferably from between 2 and 20, more preferably from between 10 and 60, and even more preferably from between 50 and 100 isolated samples, e.g. selected from the group consisting of healthy subjects, subjects having breast cancer, and subjects known as non-responders to the therapeutic treatment of breast cancer. The method of the present invention can be carried out in combination with other methods for diagnosing triple-negative breast cancer in a patient to increase the overall sensitivity and/or specificity. The determination of the level of the miRNAs of the present invention allows a diagnosis of triple-negative breast cancer in a patient.

The term "AUC", as used herein, relates to an abbreviation for the area under a curve. In particular, it refers to the area under a Receiver Operating Characteristic (ROC) curve. The term "Receiver Operating Characteristic (ROC) curve", as used herein, refers to a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve is a measure for the accuracy of a diagnostic test (the larger the area the better, optimum is 1, a random test would have a ROC curve lying on the diagonal with an area of 0.5 (see, for reference, for example, JP. Egan. Signal Detection Theory and ROC Analysis).

In the context of the present invention, the term "kit of parts (in short: kit)" is understood to be any combination of at least some of the components identified herein, which are combined, coexisting spatially, to a functional unit, and which can contain further components.

### Embodiments of the invention

The present invention will now be further described. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous, unless clearly indicated to the contrary.

The present inventors analysed miRNA expression profiles of early stage breast cancer patients compared to healthy controls. They identified single miRNAs which predict breast cancer with a high specificity, sensitivity, and accuracy. The present inventors also pursued a multiple biomarker strategy by implementing sets of miRNA biomarkers for the diagnosis of breast cancer. This approach could further increase specificity, sensitivity, and accuracy and, thus, the predictive power. In addition, the present inventors identified a specific sample type, namely a blood cellular fraction comprising erythrocytes, leukocytes, and thrombocytes, as a special source of miRNAs having a high diagnostic potential.

Thus, in a first aspect, the present invention relates to a method for diagnosing triple-negative breast cancer (TNBC) in a patient comprising the step of:
determining the level of at least one miRNA (e.g. 1, 2, 3, 4, 5, 6, 7, 8, 9, 10, 11, 12, 13, 14, 15, 16, 17, 18, 19, 20, 21, 22, or 23 miRNA(s)) representative for triple-negative breast cancer in a blood cellular fraction (particularly comprising or consisting of erythrocytes, leukocytes, and thrombocytes) from the patient,
wherein the at least one miRNA (e.g. 1, 2, 3, 4, 5, 6, 7, 8, 9, 10, 11, 12, 13, 14, 15, 16, 17, 18, 19, 20, 21, 22, or 23 miRNA(s)) is selected from the group consisting of SEQ ID NO: 1 to SEQ ID NO: 23, and a sequence having at least 80%, more preferably at least 85%, even more preferably at least 90%, and most preferably at least 95%, i.e. 80, 81, 82, 83, 84, 85, 86, 87, 88, 89, 90, 91, 92, 93, 94, 95, 96, 97, 98, or 99%, sequence identity thereto.

In one embodiment, the level of the at least one miRNA is compared to a reference level of said at least one miRNA. Thus, in one particular embodiment, the present invention relates to a method for diagnosing triple-negative breast cancer in a patient comprising the steps of:
(i) determining the level of at least one miRNA (e.g. 1, 2, 3, 4, 5, 6, 7, 8, 9, 10, 11, 12, 13, 14, 15, 16, 17, 18, 19, 20, 21, 22, or 23 miRNA(s)) representative for triple-negative breast cancer in a blood cellular fraction (particularly comprising erythrocytes, leukocytes, and thrombocytes) from the patient,
   wherein the at least one miRNA (e.g. 1, 2, 3, 4, 5, 6, 7, 8, 9, 10, 11, 12, 13, 14, 15, 16, 17, 18, 19, 20, 21, 22, or 23 miRNA(s)) is selected from the group consisting of SEQ ID NO: 1 to SEQ ID NO: 23, and a sequence having at least 80%, more preferably at least 85%, even more preferably at least 90%, and most preferably at least 95%, i.e. 80, 81, 82, 83, 84, 85, 86, 87, 88, 89, 90, 91, 92, 93, 94, 95, 96, 97, 98, or 99%, sequence identity thereto, and
(ii) comparing the level of the at least one miRNA to a reference level of said at least one miRNA.
The above comparison allows for the diagnosis of triple-negative breast cancer in the patient.

The reference level may be any level which allows to determine whether a patient suffers from triple-negative breast cancer or not. It is preferred that the reference level is the level determined by measuring at least one reference blood cellular fraction from at least one healthy subject. Preferably said reference blood cellular fractions are at least two reference blood cellular fractions, more preferably at least 2 to 100 reference blood cellular fractions, even more preferably at least 10 to 500 reference blood cellular fractions, and most preferably at least 50 to 10.000 reference blood cellular fractions, e.g. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, 1.000, 2.000, 3.000, 4.000, 5.000, or 10.000 reference blood cellular fractions. Preferably, said healthy subjects are at least two healthy subjects, more preferably at least 2 to 100 healthy subjects, even more preferably at least 10 to 500 healthy subjects, and most preferably at least 50 to 10.000 healthy subjects, e.g. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, 1.000, 2.000, 3.000, 4.000, 5.000, or 10.000 healthy subjects. It is practicable to take one reference blood cellular fraction per subject for analysis. If additional reference blood cellular fractions are required, e.g. to determine the reference level in different reference blood cellular fractions, the same subject may be (re)tested. Said reference level may be an average reference level. It may be determined by measuring reference levels and calculating the "average" value (e.g. mean, median or modal value) thereof. It is preferred that the reference blood cellular fraction is from the same source (e.g. a blood cellular fraction comprising erythrocytes, leukocytes, and thrombocytes) than the blood cellular fraction taken from the patient. It is further preferred that the reference level is obtained from a subject of the same gender (e.g. female or male) and/or of a similar age/phase of life (e.g. adults or elderly) than the patient to be tested or diagnosed.

As mentioned above, the level of the at least one miRNA is compared to a reference level of said at least one miRNA. Said reference level is the level determined by measuring a reference blood cellular fraction. For example, if the level of the miRNA according to SEQ ID NO: 1 is determined in a blood cellular fraction from a patient, it is compared to a reference level of the miRNA according to SEQ ID NO: 1 determined in a reference blood cellular fraction. Alternatively, if the level of the miRNA according to SEQ ID NO: 1 and the level of the miRNA according to SEQ ID NO: 2 is determined in a blood cellular fraction from a patient, both levels are compared to the respective reference levels, i.e. the level of the miRNA according to SEQ ID NO: 1 is compared to the reference level of the miRNA according to SEQ ID NO: 1 and the level of the miRNA according to SEQ ID NO: 2 is compared to the reference level of the miRNA according to SEQ ID NO: 2 determined in a reference blood cellular fraction.

It is further preferred that the level of the at least one miRNA selected from the group consisting of SEQ ID NO: 1 to SEQ ID NO: 6, SEQ ID NO: 8 to SEQ ID NO: 14, and SEQ ID NO: 18 to SEQ ID NO: 23 is above the reference level, which indicates that the patient has triple-negative breast cancer, and/or
the level of the at least one miRNA selected from the group consisting of SEQ ID NO: 7 and SEQ ID NO: 15 to SEQ ID NO: 17 is below the reference level, which indicates that the patient has triple-negative breast cancer.
Preferably, the level of the at least one miRNA is at least 0.4 fold or at least 1.2 fold above/below the reference level, more preferably at least 1.5-fold or at least 2-fold above/below the reference level, even more preferably at least 2.5-fold or at least 3-fold above/below the reference level, and most preferably at least 4.0-fold above/below the reference level.
In particular, with respect to the miRNA according to SEQ ID NO: 1, it is preferred that the level is at least 1.2 fold above the reference level. It is more preferred that the level is at least 1.5-fold above the reference level. It is even more preferred that the level is at least 2-fold above the reference level. It is most preferred that the level is at least 2.5-fold above the reference level.
With respect to the miRNA according to SEQ ID NO: 2, it is preferred that the level is at least 1.2 fold above the reference level. It is more preferred that the level is at least 1.5-fold above the reference level. It is even more preferred that the level is at least 2-fold above the reference level.
With respect to the miRNA according to SEQ ID NO: 3, it is preferred that the level is at least 1.2 fold above the reference level. It is more preferred that the level is at least 1.5-fold above the reference level. It is even more preferred that the level is at least 2-fold above the reference level. It is most preferred that the level is at least 3.0-fold above the reference level.
With respect to the miRNA according to SEQ ID NO: 4, it is preferred that the level is at least 1.2 fold above the reference level. It is more preferred that the level is at least 1.5-fold above the reference level. It is even more preferred that the level is at least 3.0-fold above the reference level. It is most preferred that the level is at least 4.0-fold above the reference level.
With respect to the miRNA according to SEQ ID NO: 5, it is preferred that the level is at least 1.2 fold above the reference level. It is more preferred that the level is at least 1.5-fold above the reference level. It is even more preferred that the level is at least 2.0-fold above the reference level. It is most preferred that the level is at least 2.5-fold above the reference level.
With respect to the miRNA according to SEQ ID NO: 6, it is preferred that the level is at least 1.2 fold above the reference level. It is more preferred that the level is at least 1.5-fold above the reference level. It is even more preferred that the level is at least 1.8-fold above the reference level.
With respect to the miRNA according to SEQ ID NO: 7, it is preferred that the level is at least 0.4 fold below the reference level.
With respect to the miRNA according to SEQ ID NO: 8, it is preferred that the level is at least 1.2 fold above the reference level. It is more preferred that the level is at least 1.5-fold above the reference level. It is even more preferred that the level is at least 2.5-fold above the reference level. It is most preferred that the level is at least 3.0-fold above the reference level.
With respect to the miRNA according to SEQ ID NO: 9, it is preferred that the level is at least 1.2 fold above the reference level. It is more preferred that the level is at least 1.5-fold above the reference level. It is even more preferred that the level is at least 1.8-fold above the reference level.
With respect to the miRNA according to SEQ ID NO: 10, it is preferred that the level is at least 1.2 fold above the reference level. It is more preferred that the level is at least 1.5-fold above the reference level.
With respect to the miRNA according to SEQ ID NO: 11, it is preferred that the level is at least 1.2 fold above the reference level. It is more preferred that the level is at least 1.5-fold above the reference level.
With respect to the miRNA according to SEQ ID NO: 12, it is preferred that the level is at least 1.2 fold above the reference level. It is more preferred that the level is at least 1.5-fold above the reference level. It is even more preferred that the level is at least 2.0-fold above the reference level.
With respect to the miRNA according to SEQ ID NO: 13, it is preferred that the level is at least 1.2 fold above the reference level. It is more preferred that the level is at least 1.5-fold above the reference level.
With respect to the miRNA according to SEQ ID NO: 14, it is preferred that the level is at least 1.2 fold above the reference level. It is more preferred that the level is at least 1.5-fold above the reference level.
With respect to the miRNA according to SEQ ID NO: 15, it is preferred that the level is at least 0.4 fold below the reference level.
With respect to the miRNA according to SEQ ID NO: 16, it is preferred that the level is at least 0.5 fold below the reference level.
With respect to the miRNA according to SEQ ID NO: 17, it is preferred that the level is at least 0.5 fold below the reference level.
With respect to the miRNA according to SEQ ID NO: 18, it is preferred that the level is at least 1.2 fold above the reference level. It is more preferred that the level is at least 1.5-fold above the reference level. It is even more preferred that the level is at least 2.0-fold above the reference level.
With respect to the miRNA according to SEQ ID NO: 19, it is preferred that the level is at least 1.2 fold above the reference level. It is more preferred that the level is at least 1.5-fold above the reference level. It is even more preferred that the level is at least 2.0-fold above the reference level.
With respect to the miRNA according to SEQ ID NO: 20, it is preferred that the level is at least 1.2 fold above the reference level. It is more preferred that the level is at least 1.5-fold above the reference level. It is even more preferred that the level is at least 2.0-fold above the reference level.
With respect to the miRNA according to SEQ ID NO: 21, it is preferred that the level is at least 1.2 fold above the reference level. It is more preferred that the level is at least 1.5-fold above the reference level.
With respect to the miRNA according to SEQ ID NO: 22, it is preferred that the level is at least 1.2 fold above the reference level. It is more preferred that the level is at least 1.5-fold above the reference level.
With respect to the miRNA according to SEQ ID NO: 23, it is preferred that the level is at least 1.2 fold above the reference level. It is more preferred that the level is at least 1.5-fold above the reference level.

Alternatively or additionally, it is preferred that the reference level is the level determined by measuring at least one reference blood cellular fraction from at least one subject known to have triple-negative breast cancer. Preferably said reference blood cellular fractions are at least two reference blood cellular fractions, more preferably at least 2 to 100 reference blood cellular fractions, even more preferably at least 10 to 500 reference blood cellular fractions, and most preferably at least 50 to 10.000 reference blood cellular fractions, e.g. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, 1.000, 2.000, 3.000, 4.000, 5.000, or 10.000 reference blood cellular fractions. Preferably, said subjects having triple-negative breast cancer are at least two subjects having triple-negative breast cancer, more preferably at least 2 to 100 subjects having triple-negative breast cancer, even more preferably at least 10 to 500 subjects having triple-negative breast cancer, and most preferably at least 50 to 10.000 subjects having triple-negative breast cancer, e.g. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, 1.000, 2.000, 3.000, 4.000, 5.000, or 10.000 subjects having triple-negative breast cancer. It is practicable to take one reference blood cellular fraction per subject for analysis. If additional reference blood cellular fractions are required, e.g. to determine the reference level in different reference blood cellular fractions, the same subject may be (re)tested. Said reference level may be an average reference level. It may be determined by measuring reference levels and calculating the "average" value (e.g. mean, median or modal value) thereof. It is preferred that the reference blood cellular fraction is from the same source (e.g. a blood cellular fraction comprising erythrocytes, leukocytes, and thrombocytes) than the blood cellular fraction taken from the patient. It is further preferred that the reference level is obtained from a subject of the same gender (e.g. female or male) and/or of a similar age/phase of life (e.g. adults or elderly) than the patient to be tested.

In an alternative embodiment, an algorithm or a mathematical function is applied to the level of the at least one miRNA. Thus, in an alternative embodiment, the present invention relates to a method for diagnosing triple-negative breast cancer (TNBC) in a patient comprising the steps of:
(i) determining the level of at least one miRNA (e.g. 1, 2, 3, 4, 5, 6, 7, 8, 9, 10, 11, 12, 13, 14, 15, 16, 17, 18, 19, 20, 21, 22, or 23 miRNA(s)) representative for triple-negative breast cancer in a blood cellular fraction (particularly comprising erythrocytes, leukocytes, and thrombocytes) from the patient,
   wherein the at least one miRNA (e.g. 1, 2, 3, 4, 5, 6, 7, 8, 9, 10, 11, 12, 13, 14, 15, 16, 17, 18, 19, 20, 21, 22, or 23 miRNA(s)) is selected from the group consisting of SEQ ID NO: 1 to SEQ ID NO: 23, and a sequence having at least 80%, more preferably at least 85%, even more preferably at least 90%, and most preferably at least 95%, i.e. 80, 81, 82, 83, 84, 85, 86, 87, 88, 89, 90, 91, 92, 93, 94, 95, 96, 97, 98, or 99%, sequence identity thereto, and
(ii) applying an algorithm or mathematical function to the level of said at least one miRNA. The above application allows to diagnose triple-negative breast cancer in the patient.

The algorithm or mathematical function may be any algorithm or mathematical function which allows to decide if triple-negative breast cancer is present in the patient or not. It is preferred that the algorithm or mathematical function is obtained from a reference level of at least one miRNA determined in at least one blood cellular fraction from at least one subject having triple-negative breast cancer, and
a reference level of at least one miRNA determined in at least one blood cellular fraction from at least one healthy subject.
Preferably, said reference blood cellular fractions are at least two reference blood cellular fractions, more preferably at least 2 to 100 reference blood cellular fractions, even more preferably at least 10 to 500 reference blood cellular fractions, and most preferably at least 50 to 10.000 reference blood cellular fractions, e.g. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, 1.000, 2.000, 3.000, 4.000, 5.000, or 10.000 reference blood cellular fractions. Preferably, said healthy subjects are at least two healthy subjects, more preferably at least 2 to 100 healthy subjects, even more preferably at least 10 to 500 healthy subjects, and most preferably at least 50 to 10.000 healthy subjects, e.g. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, 1.000, 2.000, 3.000, 4.000, 5.000, or 10.000 healthy subjects. Preferably, said subjects having triple-negative breast cancer are at least two subjects having triple-negative breast cancer, more preferably at least 2 to 100 subjects having triple-negative breast cancer, even more preferably at least 10 to 500 subjects having triple-negative breast cancer, and most preferably at least 50 to 10.000 subjects having triple-negative breast cancer, e.g. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, 1.000, 2.000, 3.000, 4.000, 5.000, or 10.000 subjects having triple-negative breast cancer. It is practicable to take one reference blood cellular fraction per subject for analysis. If additional reference blood cellular fractions are required, e.g. to determine the reference level in different reference blood cellular fractions, the same subject may be (re)tested. Said reference level may be an average reference level. It may be determined by measuring reference levels and calculating the "average" value (e.g. mean, median or modal value) thereof. It is preferred that the reference blood cellular fraction is from the same source (e.g. a blood cellular fraction comprising erythrocytes, leukocytes, and thrombocytes) than the blood cellular fraction taken from the patient. It is further preferred that the reference level is obtained from a subject of the same gender (e.g. female or male) and/or of a similar age/phase of life (e.g. adults or elderly) than the patient to be tested or diagnosed.
It is more preferred that the algorithm or mathematical function is obtained using a machine learning approach.
It is even more preferred that the machine learning approach involves the following steps:
(i) inputting
   the reference level of at least one miRNA determined in at least one reference blood cellular fraction from at least one subject having triple-negative breast cancer, and
   the reference level of at least one miRNA determined in at least one reference blood cellular fraction from at least one healthy subject, and
(ii) computing an algorithm or a mathematical function based on said reference levels that is suitable to distinguish between triple-negative breast cancer and healthiness or to decide if triple-negative breast cancer is present in the patient or not.
The inventors of the present invention found that the application of a machine learning approach leads to the obtainment of an algorithm or a mathematical function that is trained by the reference levels mentioned above which allows a better discrimination between healthiness and triple-negative breast cancer. In this way, the performance of patient's diagnosis can be improved.
Machine learning approaches may include, but are not limited to, supervised or unsupervised analysis: classification techniques (e.g. naive Bayes, Linear Discriminant Analysis, Quadratic Discriminant Analysis Neural Nets, Tree based approaches, Support Vector Machines, Nearest Neighbour Approaches), Regression techniques (e.g. linear Regression, Multiple Regression, logistic regression, probit regression, ordinal logistic regression ordinal probit regression, Poisson Regression, negative binomial Regression, multinomial logistic Regression, truncated regression), Clustering techniques (e.g. k-means clustering, hierarchical clustering, PCA), Adaptations, extensions, and combinations of the previously mentioned approaches.
In particular, support vector machines (SVMs) are a set of related supervised learning methods which are preferably used for classification and regression. For example, given a set of training examples, each marked as belonging to one of two categories (e.g. diseased, i.e. suffering from triple-negative breast cancer, or healthy, i.e. not suffering from triple-negative breast cancer), an SVM algorithm builds a model that predicts whether a new example (e.g. sample to be tested) falls into one category or the other (e.g. diseased, i.e. suffering from triple-negative breast cancer, or healthy, i.e. not suffering from triple-negative breast cancer). A SVM model is a representation of the training examples as points in space, mapped so that the training examples of the separate categories (e.g. diseased, i.e. suffering from triple-negative breast cancer, or healthy, i.e. not suffering triple-negative breast cancer) are divided by a clear gap that is as wide as possible. New examples (e.g. samples to be tested) are then mapped into that same space and predicted to belong to a category based on which side of the gap they fall on (e.g. diseased, i.e. suffering from triple-negative breast cancer, or healthy, i.e. not suffering from triple-negative breast cancer). More formally, a support vector machine constructs a hyperplane or set of hyperplanes in a high or infinite dimensional space, which can be used for classification, regression or other tasks. A good separation is achieved by the hyperplane that has the largest distance to the nearest training data points of any class (so-called functional margin), since in general the larger the margin the lower the generalization error of the classifier.

It is preferred that the blood cellular fraction comprises erythrocytes, leukocytes, and thrombocytes. As mentioned above, the blood cellular fraction is produced from whole blood by removing the extracellular fraction (serum and/or plasma). In other words, the blood cellular fraction is depleted of the extracellular blood components, such as serum and/or plasma.

As mentioned above, the method for diagnosing triple-negative breast cancer in a patient comprises the step of:
determining the level of at least one miRNA representative for triple-negative breast cancer in a blood cellular fraction from the patient.
It is preferred that the at least one miRNA is selected from the group consisting of SEQ ID NO: 1 to SEQ ID NO: 8, and a sequence having at least 80% sequence identity thereto.
For example, the level of the miRNA according to SEQ ID NO: 1, SEQ ID NO: 2, SEQ ID NO: 3, SEQ ID NO: 4, SEQ ID NO: 5, SEQ ID NO: 6, SEQ ID NO: 7, or SEQ ID NO: 8 is determined.
More specifically, the at least one miRNA is selected from the group consisting of SEQ ID NO: 1 to SEQ ID NO: 6, SEQ ID NO: 8, and a sequence having at least 80% sequence identity thereto, or the at least one miRNA is selected from the group consisting of SEQ ID NO: 2 to SEQ ID NO: 8, and a sequence having at least 80% sequence identity thereto.
For example,
(i) the level of the miRNA according to SEQ ID NO: 2 and the level of at least one further miRNA selected from the group consisting of SEQ ID NO: 3 to SEQ ID NO: 8,
(ii) the level of the miRNA according to SEQ ID NO: 3 and the level of at least one further miRNA selected from the group consisting of SEQ ID NO: 2 and SEQ ID NO: 4 to SEQ ID NO: 8,
(iii) the level of the miRNA according to SEQ ID NO: 4 and the level of at least one further miRNA selected from the group consisting of SEQ ID NO: 2, SEQ ID NO: 3, and SEQ ID NO: 5 to SEQ ID NO: 8,
(iv) the level of the miRNA according to SEQ ID NO: 5 and the level of at least one further miRNA selected from the group consisting of SEQ ID NO: 2 to SEQ ID NO: 4 and SEQ ID NO: 6 to SEQ ID NO: 8,
(v) the level of the miRNA according to SEQ ID NO: 6 and the level of at least one further miRNA selected from the group consisting of SEQ ID NO: 2 to SEQ ID NO: 5, SEQ ID NO: 7, and SEQ ID NO: 8,
(vi) the level of the miRNA according to SEQ ID NO: 7 and the level of at least one further miRNA selected from the group consisting of SEQ ID NO: 2 to SEQ ID NO: 6 and SEQ ID NO: 8, and
(vii) the level of the miRNA according to SEQ ID NO: 8 and the level of at least one further miRNA selected from the group consisting of SEQ ID NO: 2 to SEQ ID NO: 7
is determined.
Further, for example,
(i) the level of the miRNAs according to SEQ ID NO: 2 and SEQ ID NO: 3,
(ii) the level of the miRNAs according to SEQ ID NO: 2, SEQ ID NO: 3, and SEQ ID NO: 4,
(iii) the level of the miRNAs according to SEQ ID NO: 2, SEQ ID NO: 3, SEQ ID NO: 4, and SEQ ID NO: 5,
(iv) the level of the miRNAs according to SEQ ID NO: 2, SEQ ID NO: 3, SEQ ID NO: 4, SEQ ID NO: 5, and SEQ ID NO: 6,
(v) the level of the miRNAs according to SEQ ID NO: 2, SEQ ID NO: 3, SEQ ID NO: 4, SEQ ID NO: 5, SEQ ID NO: 6, and SEQ ID NO: 7, and
(vi) the level of the miRNAs according to SEQ ID NO: 2, SEQ ID NO: 3, SEQ ID NO: 4, SEQ ID NO: 5, SEQ ID NO: 6, SEQ ID NO: 7, and SEQ ID NO: 8
is determined.
More specifically, the at least one miRNA is selected from the group consisting of SEQ ID NO: 3 to SEQ ID NO: 5, SEQ ID NO: 8, and a sequence having at least 80% sequence identity thereto.
For example,
(i) the level of the miRNAs according to SEQ ID NO: 3 and SEQ ID NO: 4,
(ii) the level of the miRNAs according to SEQ ID NO: 3 and SEQ ID NO: 5,
(iii) the level of the miRNAs according to SEQ ID NO: 3 and SEQ ID NO: 8,
(iv) the level of the miRNAs according to SEQ ID NO: 4 and SEQ ID NO: 5,
(v) the level of the miRNAs according to SEQ ID NO: 4 and SEQ ID NO: 8,
(vi) the level of the miRNAs according to SEQ ID NO: 5 and SEQ ID NO: 8,
(vii) the level of the miRNAs according to SEQ ID NO: 3, SEQ ID NO: 4, and SEQ ID NO: 5,
(viii) the level of the miRNAs according to SEQ ID NO: 3, SEQ ID NO: 4, and SEQ ID NO: 8,
(ix) the level of the miRNAs according to SEQ ID NO: 3, SEQ ID NO: 5, and SEQ ID NO: 8,
(x) the level of the miRNAs according to SEQ ID NO: 4, SEQ ID NO: 5, and SEQ ID NO: 8, and
(xi) the level of the miRNAs according to SEQ ID NO: 3, SEQ ID NO: 4, SEQ ID NO: 5, and SEQ ID NO: 8
is determined.

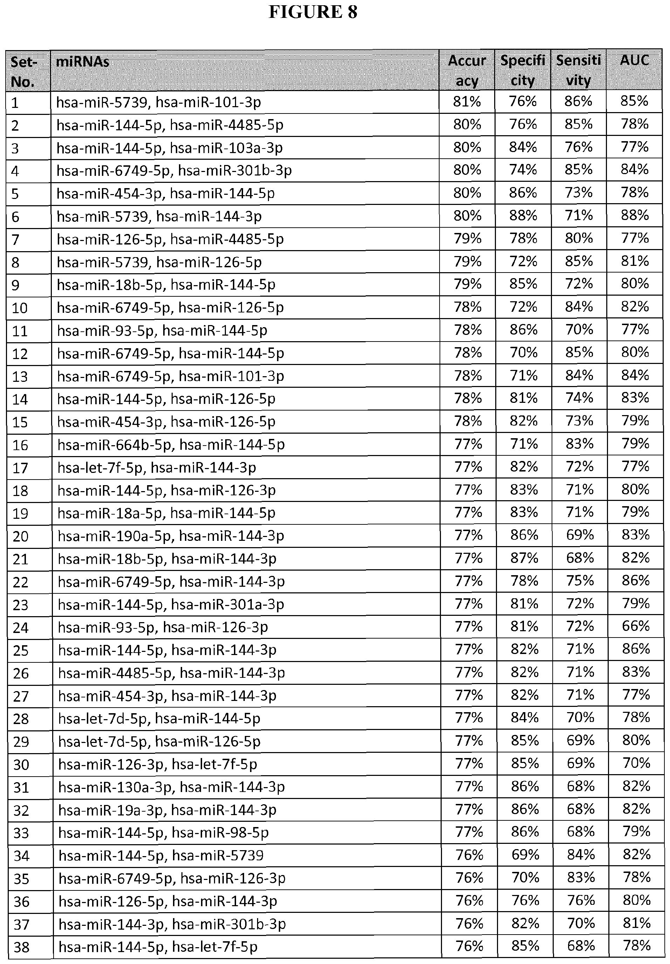

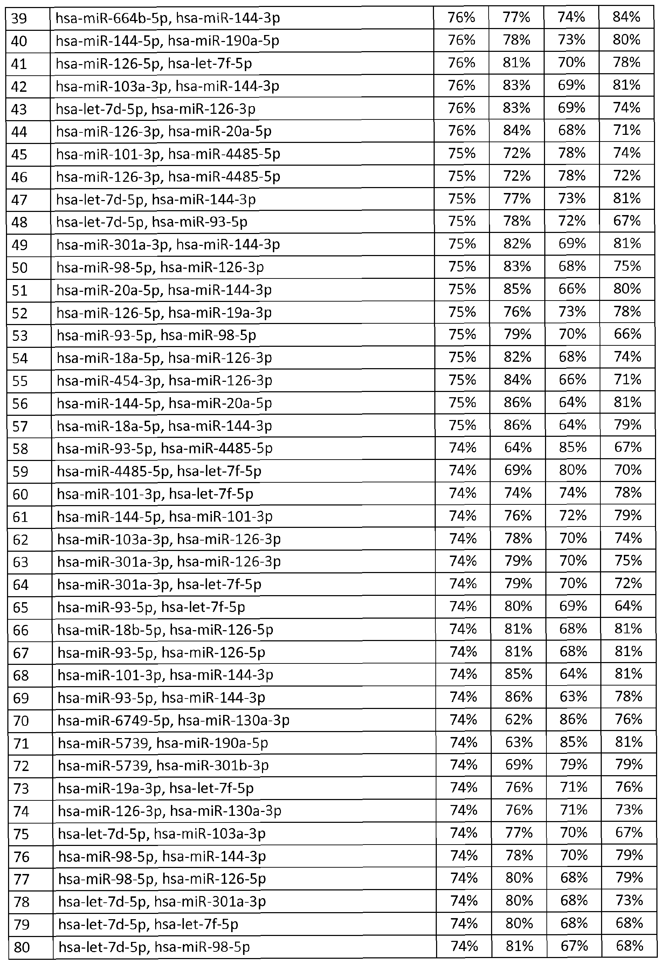

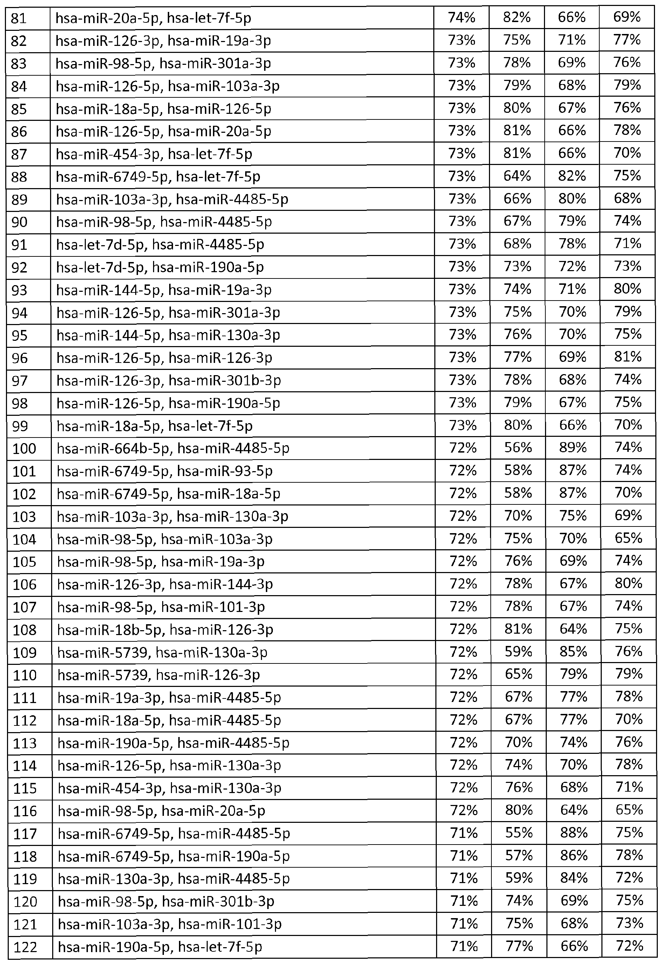

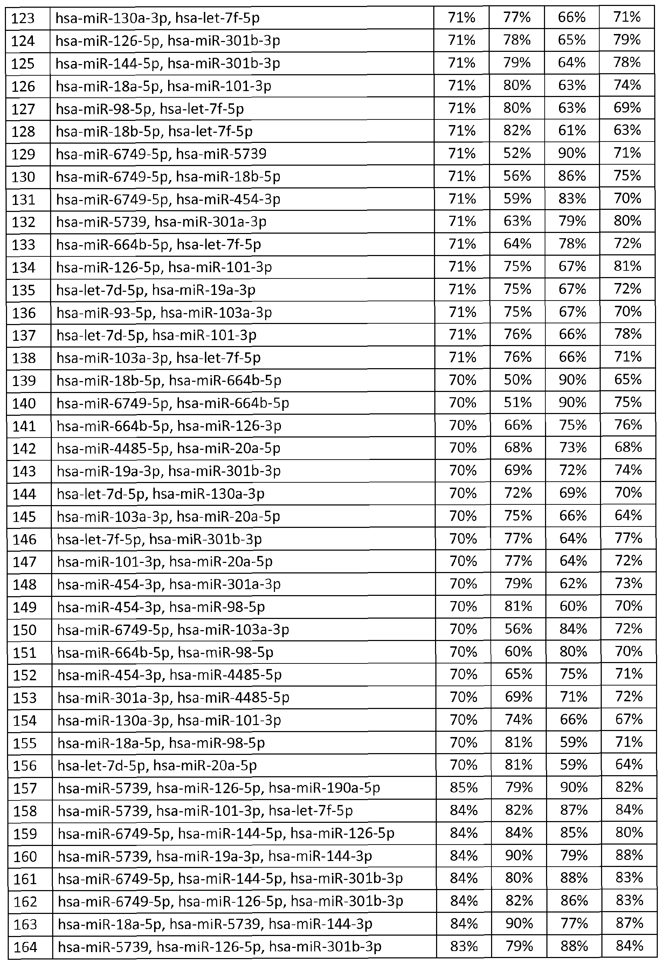

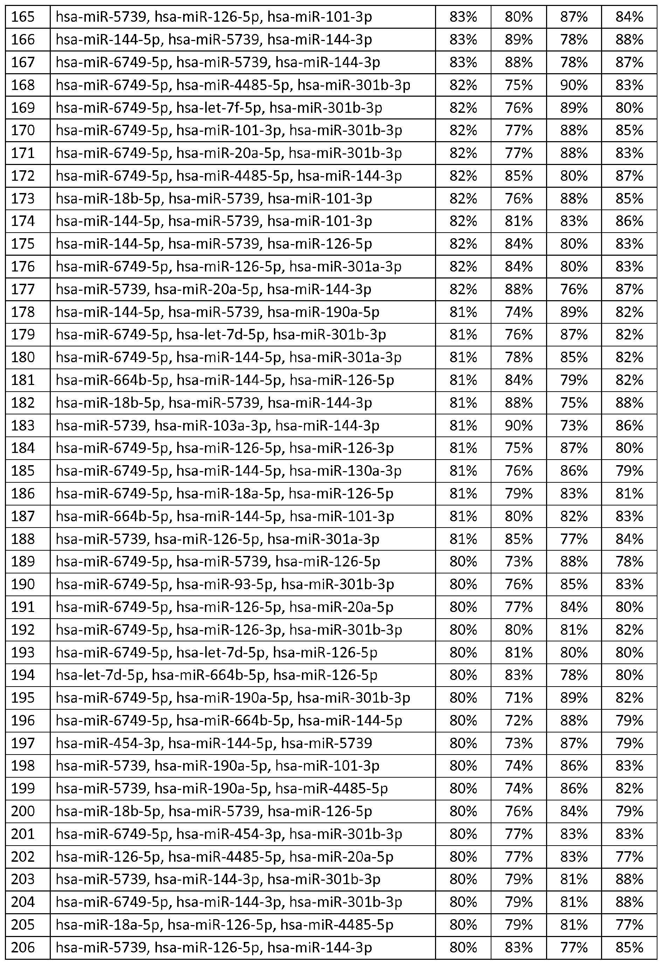

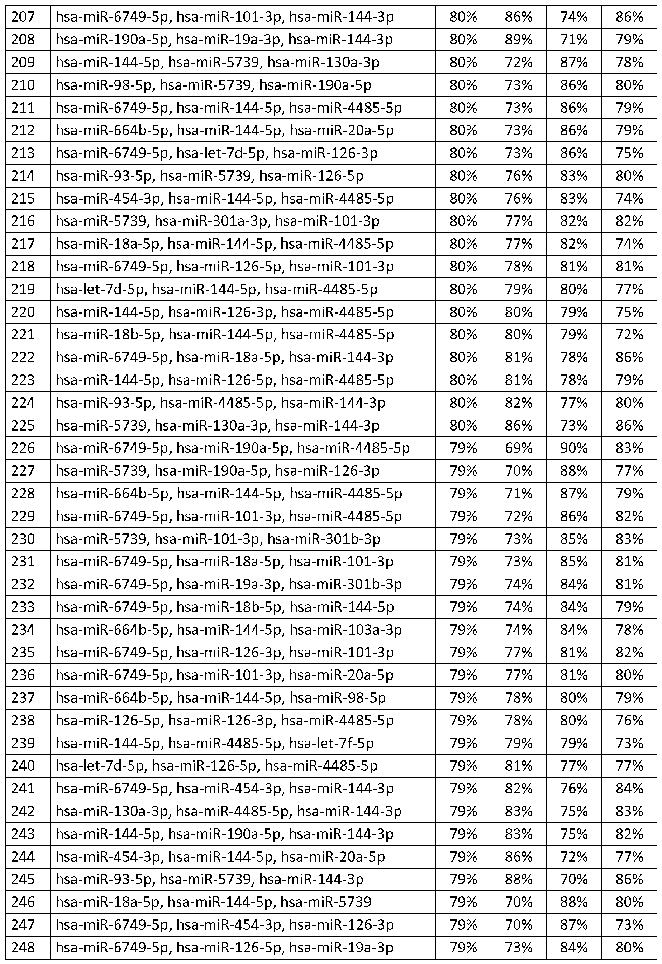

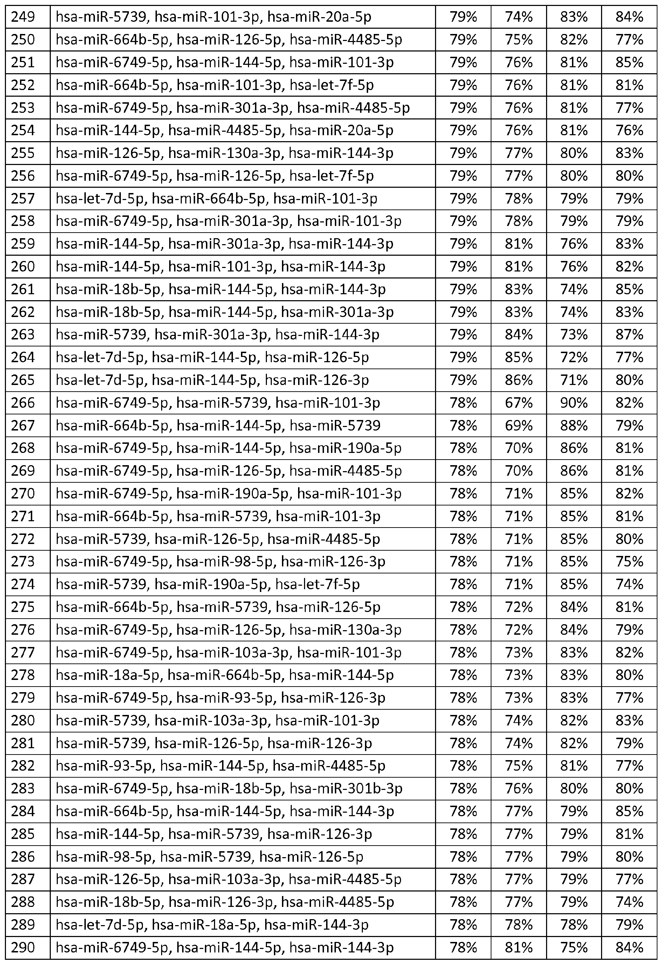

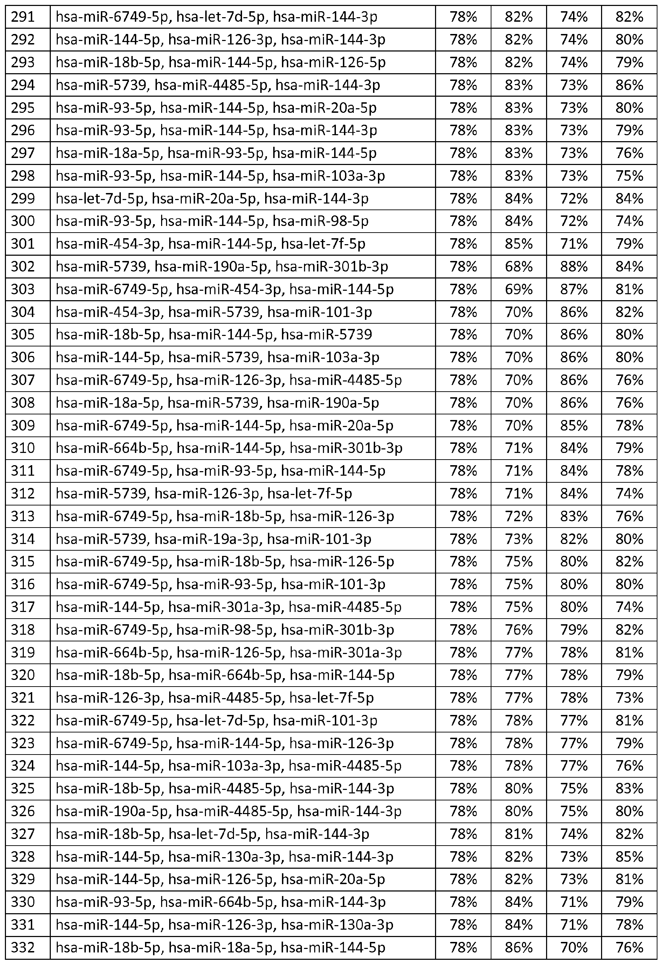

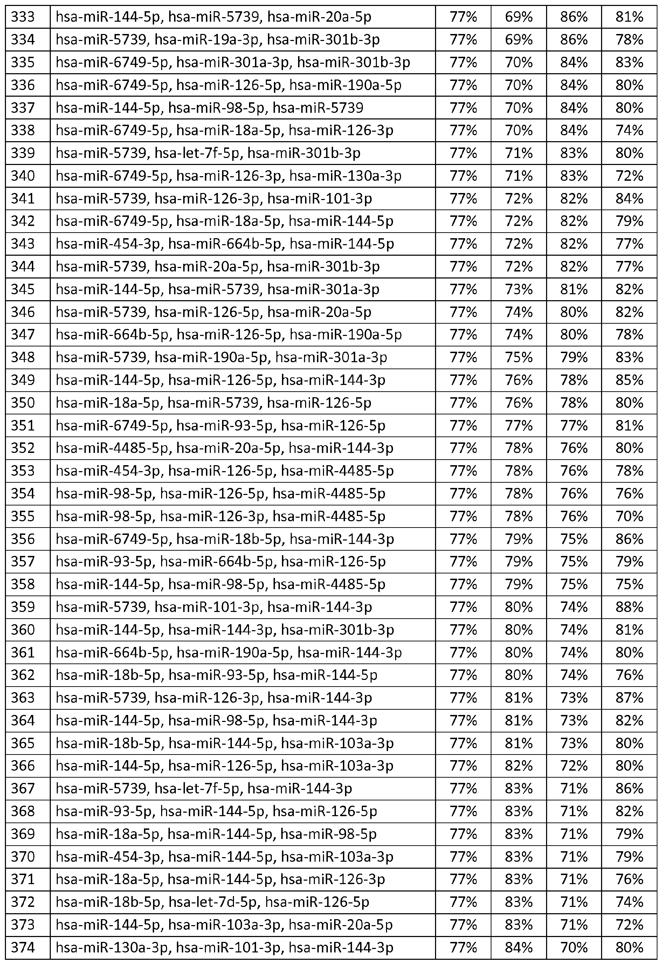

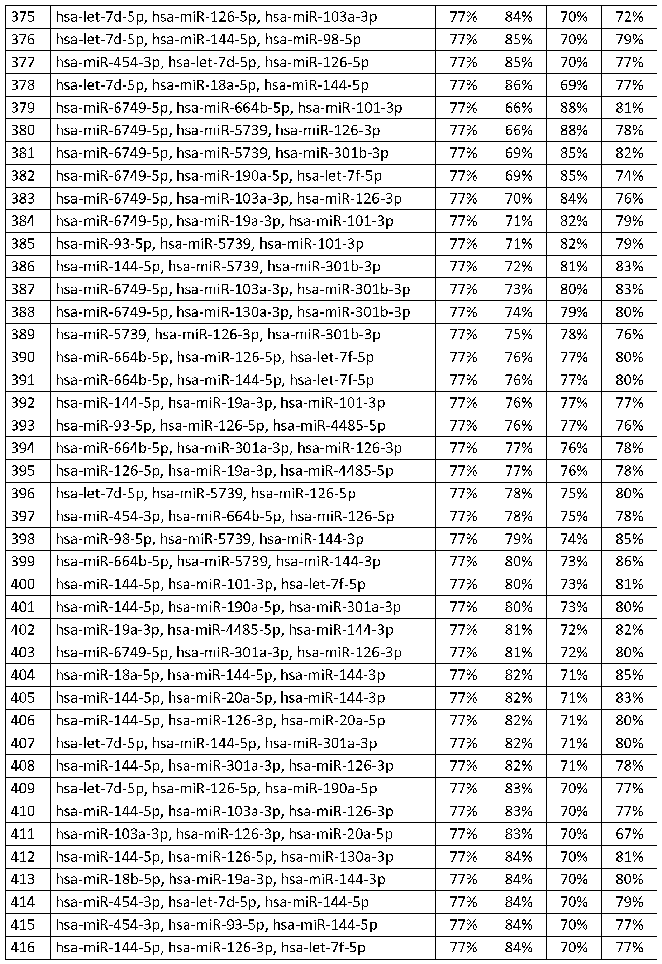

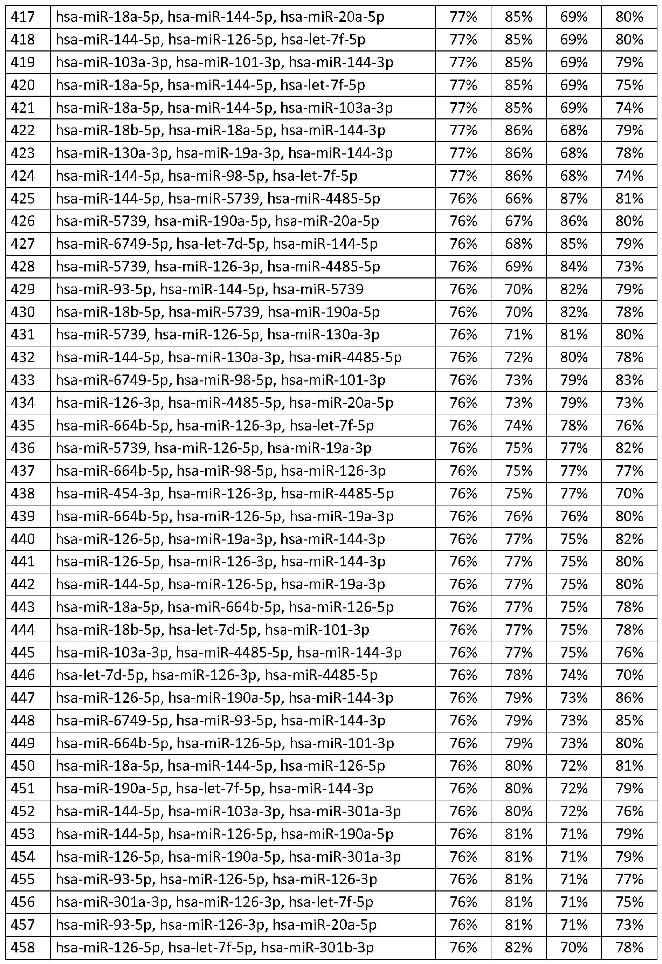

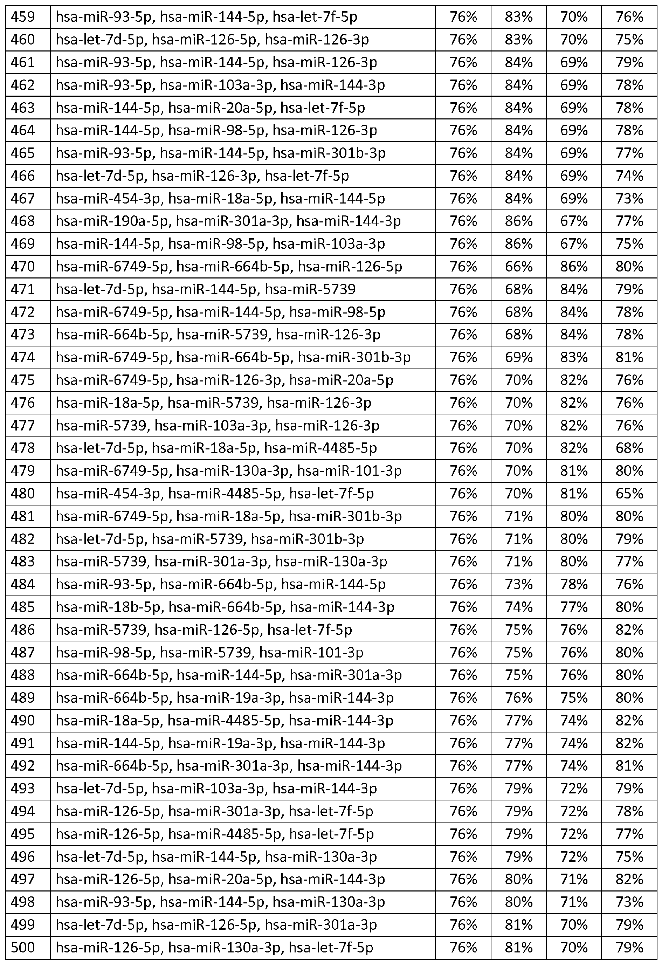

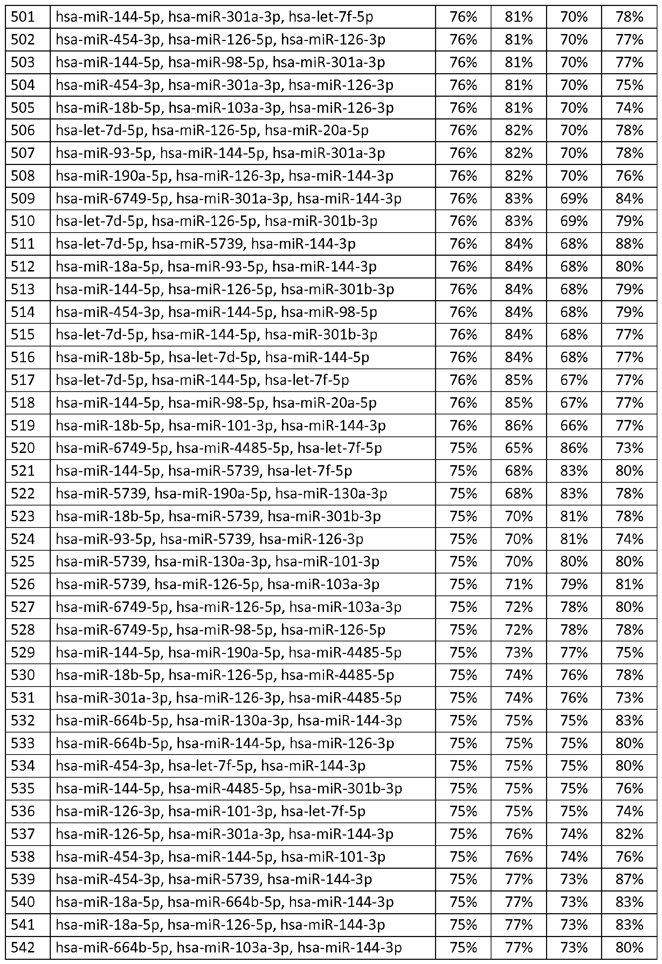

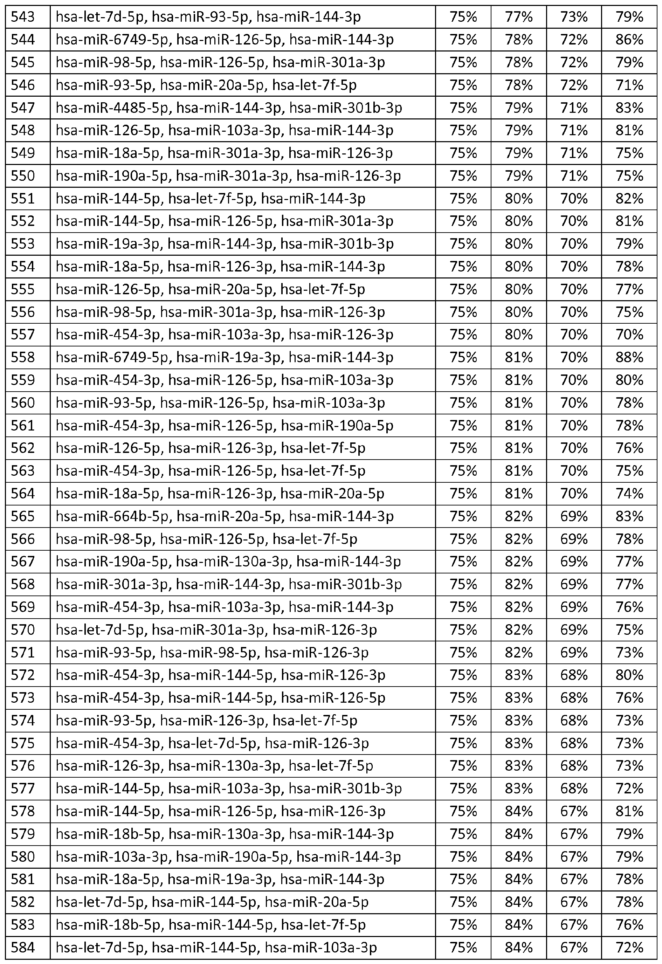

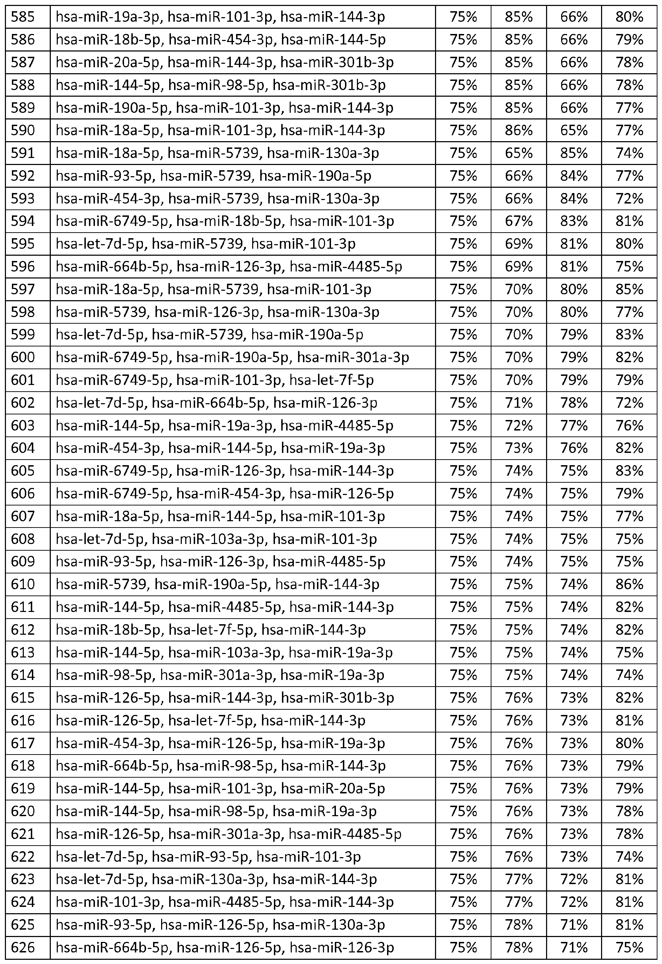

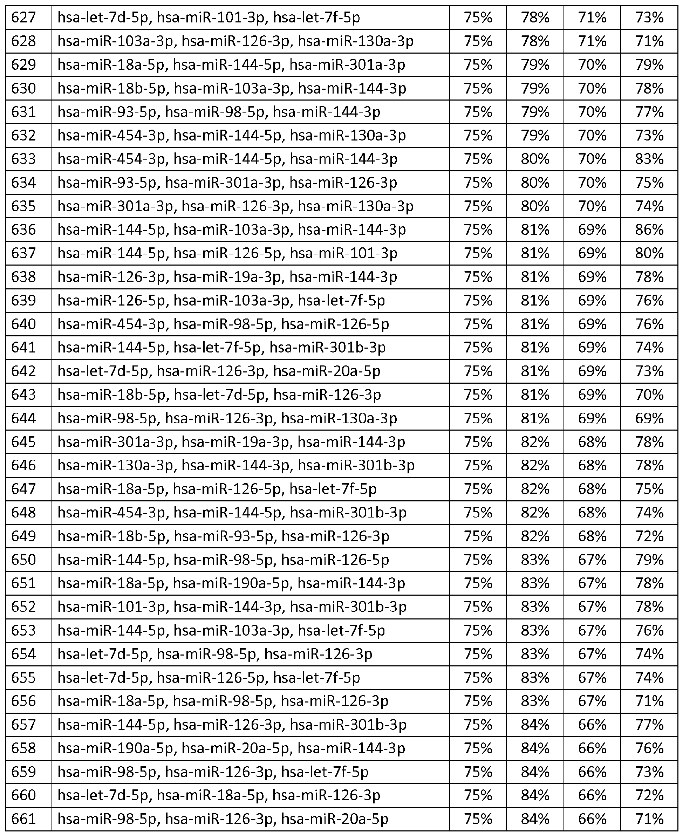

It is more preferred that the at least one miRNA are one or more sets of miRNAs, wherein the one or more sets of miRNAs are listed in Figure 8.

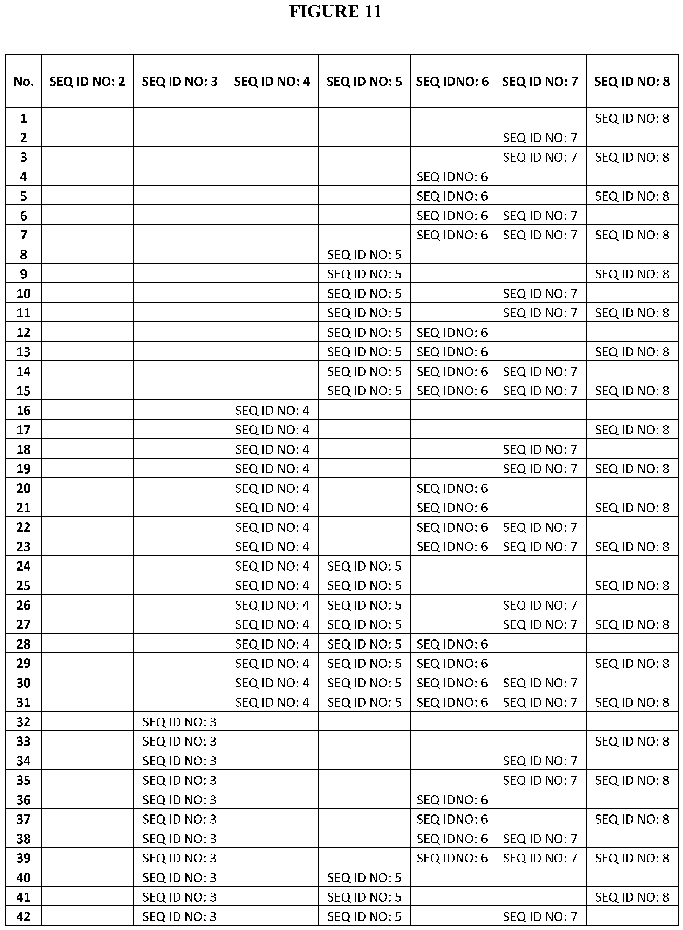

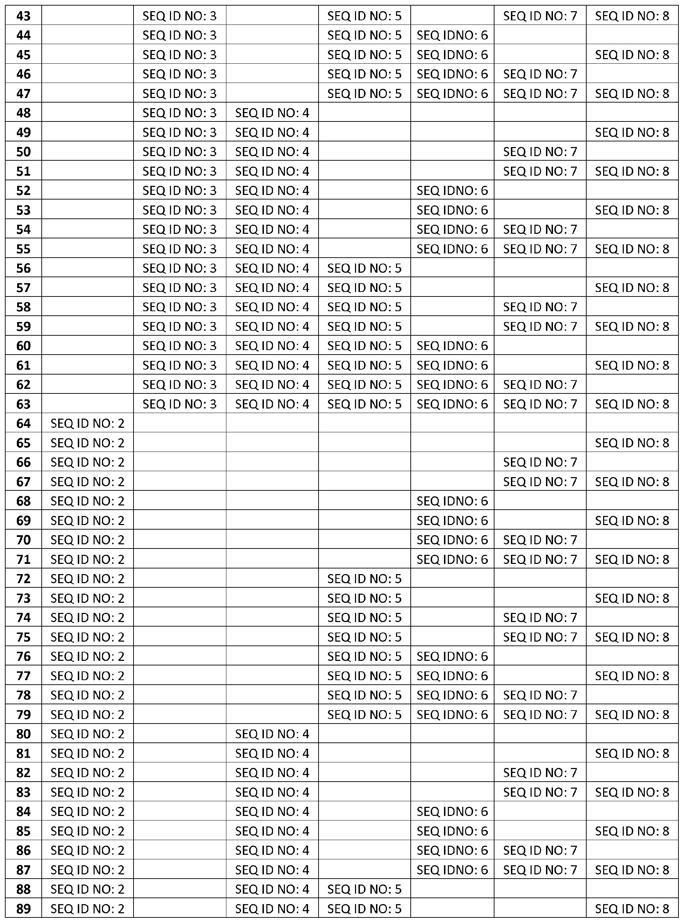

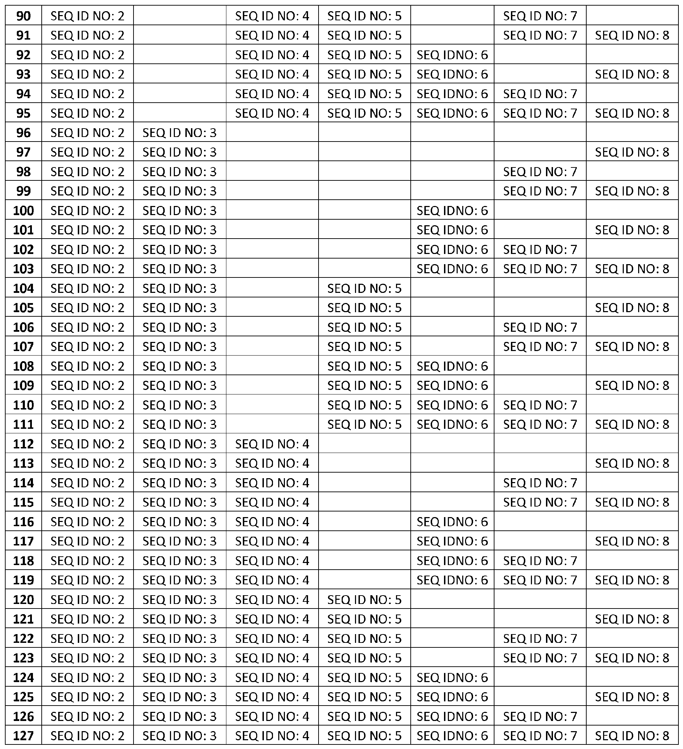

All combinations of 2, 3, 4, 5, 6, or 7 miRNAs selected from the group consisting of SEQ ID NO: 2 to SEQ ID NO: 8 as well as the single miRNAs which level can be determined are comprised in FIGURE 11.

In the method for diagnosing triple-negative breast cancer in a patient, the level of the at least one miRNA selected from the group consisting of SEQ ID NO: 1 to SEQ ID NO: 23, and a sequence having at least 80% sequence identity thereto is determined, wherein the diagnosis allows to differentiate between patients having triple-negative breast cancer and patients not having triple-negative breast cancer. Specifically, the at least one miRNA is SEQ ID NO: 22 or a sequence having at least 80% sequence identity thereto, wherein the diagnosis allows to differentiate between patients having triple-negative breast cancer that will respond (positively) to a therapeutic treatment, preferably chemotherapy, and patients having triple-negative breast cancer that will not respond (positively) to a therapeutic treatment, preferably chemotherapy.

The determination of the level of the at least one miRNA may be carried out by any convenient means for determining the level of a nucleotide sequence such as miRNA. For this purpose, qualitative, semi-quantitative and quantitative detection methods can be used. Quantitative detection methods are preferred. A variety of techniques are well known to the person skilled in the art. It is preferred that the level of the at least one miRNA representative for breast cancer in a blood sample from a patient is determined by nucleic acid hybridization, nucleic acid amplification, polymerase extension, sequencing, mass spectroscopy, or any combination thereof.
Nucleic acid amplification, for example, may be performed using real time polymerase chain reaction (RT-PCR) such as real time quantitative PCR (RT qPCR). This technique is suitable for detecting the level of a single miRNA. It is particularly suitable for detecting low abandoned miRNAs. However, real time quantitative PCR (RT qPCR) also allows the analysis of multiple miRNAs comprised in a blood sample from a patient.
The aforesaid real time polymerase chain reaction (RT-PCR) may include the following steps: (i) extracting total RNA from the blood sample isolated from the patient, (ii) obtaining cDNA samples by RNA reverse transcription (RT) reaction using miRNA-specific primers, (iii) designing miRNA-specific cDNA forward primers and providing universal reverse primers to amplify the cDNA via polymerase chain reaction (PCR), (iv) adding a fluorescent probe to conduct PCR, and (v) detecting and comparing the variation in levels of miRNAs in the blood sample isolated from the patient relative to those of miRNAs in a reference blood sample isolated from a healthy subject.
A variety of kits and protocols to determine the miRNA level by real time polymerase chain reaction (RT-PCR) such as real time quantitative PCR (RT qPCR) are available. For example, reverse transcription of miRNAs may be performed using the TaqMan MicroRNA Reverse Transcription Kit (Applied Biosystems) according to manufacturer's recommendations. Briefly, miRNA may be combined with dNTPs, MultiScribe reverse transcriptase and the primer specific for the target miRNA. The resulting cDNA may be diluted and may be used for PCR reaction. The PCR may be performed according to the manufacturer's recommendation (Applied Biosystems). Briefly, cDNA may be combined with the TaqMan assay specific for the target miRNA and PCR reaction may be performed using ABI7300.
Nucleic acid hybridization, for example, may be performed using a microarray/biochip or *in situ* hybridization. The microarray/biochip allows the analysis of a single miRNA as well as multiple miRNAs comprised in a blood sample from a patient. For nucleic acid hybridization, for example, the polynucleotides (probes) described herein with complementarity to the corresponding miRNAs to be detected are attached to a solid phase to generate a microarray/biochip. Said microarray/biochip is then incubated with miRNAs, isolated (e.g. extracted) from the blood sample, which may be labelled or unlabelled. Upon hybridization of the labelled miRNAs to the complementary polynucleotide sequences on the microarray/biochip, the success of hybridisation may be controlled and the intensity of hybridization may be determined via the hybridisation signal of the label in order to determine the level of each tested miRNA in said blood sample.

Preferably, the level of the at least one miRNA which is determined is the expression level. Accordingly, it is preferred that the method for diagnosing triple-negative breast cancer in a patient comprises the step of: determining the expression level of at least one miRNA representative for triple-negative breast cancer in a blood cellular fraction from the patient.

The diagnosis of triple-negative breast cancer may comprise
(i) determining the occurrence/presence of triple-negative breast cancer,
(ii) monitoring the course of triple-negative breast cancer,
(iii) staging of triple-negative breast cancer,
(iv) measuring the response of a patient with triple-negative breast cancer to therapeutic intervention, and/or
(v) segmentation of patients suffering from triple-negative breast cancer.

In a second aspect, the present invention relates to the use of at least one polynucleotide (e.g. 1, 2, 3, 4, 5, 6, 7, 8, 9, 10, 11, 12, 13, 14, 15, 16, 17, 18, 19, 20, 21, 22, or 23 polynucleotide(s)) for detecting at least one miRNA (e.g. 1, 2, 3, 4, 5, 6, 7, 8, 9, 10, 11, 12, 13, 14, 15, 16, 17, 18, 19, 20, 21, 22, or 23 miRNA(s)) for diagnosing triple-negative breast cancer (TNBC) in a blood cellular fraction (particularly comprising or consisting of erythrocytes, leukocytes, and thrombocytes) from a patient, wherein the at least one miRNA (e.g. 1, 2, 3, 4, 5, 6, 7, 8, 9, 10, 11, 12, 13, 14, 15, 16, 17, 18, 19, 20, 21, 22, or 23 miRNA(s)) is selected from the group consisting of SEQ ID NO: 1 to SEQ ID NO: 23.

It is further preferred that
(i) the at least one polynucleotide is complementary to the at least one miRNA mentioned above, or
(ii) the at least one polynucleotide has at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity to the polynucleotide according to (i).
It is particularly preferred that the polynucleotide as defined in (ii) has at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity over a continuous stretch of at least 10, 11, 12, 13, 14, 15, 16, 17, 18, 19, 20, 21, 22, 23, 24, 25, or more nucleotides, preferably over the whole length, to the polynucleotide according to (i). In addition, the polynucleotide as defined in (ii) (i.e. polynucleotide variant) is only regarded as a polynucleotide as defined in (ii) (i.e. polynucleotide variant) within the context of the present invention, if it is still capable of binding to, hybridizing with, or detecting the respective target miRNA, i.e. the target miRNA according to SEQ ID NO: 1 to SEQ ID NO: 23, through one or more types of chemical bonds, usually through complementary base pairing, usually through hydrogen bond formation under stringent hybridization conditions. The skilled person can readily assess whether a polynucleotide as defined in (ii) (i.e. polynucleotide variant) is still capable of binding to, hybridizing with, recognizing or detecting the respective target miRNA, i.e. the target miRNA according to SEQ ID NO: 1 to SEQ ID NO: 23. Suitable assays to determine whether hybridization under stringent conditions still occurs are well known in the art. However, as an example, a suitable assay to determine whether hybridization still occurs comprises the steps of: (a) incubating the polynucleotide as defined in (ii) attached onto a biochip with the respective target miRNA, i.e. the target miRNA according to SEQ ID NO: 1 to SEQ ID NO: 23, (b) washing the biochip to remove unspecific bindings, (c) subjecting the biochip to a detection system, and (c) analyzing whether the polynucleotide can still hybridize with the respective target miRNA. As a positive control, the respective non-mutated polynucleotide as defined in (i) may be used. Preferably stringent hybridization conditions include the following: 50% formamide, 5x SSC, and 1% SDS, incubating at 42°C, or, 5x SSC, 1% SDS, incubating at 65°C, with wash in 0.2x SSC, and 0.1% SDS at 65°C; or 6x SSPE, 10 % formamide, 0.01 %,Tween 20, 0.1 x TE buffer, 0.5 mg/ml BSA, 0.1 mg/ml herring sperm DNA, incubating at 42°C with wash in 05x SSPE and 6x SSPE at 45°C.

It is preferred that the blood cellular fraction comprises erythrocytes, leukocytes, and thrombocytes. As mentioned above, the blood cellular fraction is produced from whole blood by removing the extracellular fraction (serum and/or plasma). In other words, the blood cellular fraction is depleted of the extracellular blood components, such as serum and/or plasma.

As mentioned above, at least one polynucleotide is used for detecting at least one miRNA for diagnosing triple-negative breast cancer in a blood cellular fraction from a patient. It is preferred that the at least one miRNA is selected from the group consisting of SEQ ID NO: 1 to SEQ ID NO: 8.
For example, the miRNA according to SEQ ID NO: 1, SEQ ID NO: 2, SEQ ID NO: 3, SEQ ID NO: 4, SEQ ID NO: 5, SEQ ID NO: 6, SEQ ID NO: 7, or SEQ ID NO: 8 is detected.
More specifically, the at least one miRNA is selected from the group consisting of SEQ ID NO: 1 to SEQ ID NO: 6 and SEQ ID NO: 8, or the at least one miRNA is selected from the group consisting of SEQ ID NO: 2 to SEQ ID NO: 8.
For example,
(i) the miRNA according to SEQ ID NO: 2 and at least one further miRNA selected from the group consisting of SEQ ID NO: 3 to SEQ ID NO: 8,
(ii) the miRNA according to SEQ ID NO: 3 and at least one further miRNA selected from the group consisting of SEQ ID NO: 2 and SEQ ID NO: 4 to SEQ ID NO: 8,
(iii) the miRNA according to SEQ ID NO: 4 and at least one further miRNA selected from the group consisting of SEQ ID NO: 2, SEQ ID NO: 3, and SEQ ID NO: 5 to SEQ ID NO: 8,
(iv) the miRNA according to SEQ ID NO: 5 and at least one further miRNA selected from the group consisting of SEQ ID NO: 2 to SEQ ID NO: 4 and SEQ ID NO: 6 to SEQ ID NO: 8,
(v) the miRNA according to SEQ ID NO: 6 and at least one further miRNA selected from the group consisting of SEQ ID NO: 2 to SEQ ID NO: 5, SEQ ID NO: 7, and SEQ ID NO: 8,
(vi) the miRNA according to SEQ ID NO: 7 and at least one further miRNA selected from the group consisting of SEQ ID NO: 2 to SEQ ID NO: 6 and SEQ ID NO: 8, and
(vii) the miRNA according to SEQ ID NO: 8 and at least one further miRNA selected from the group consisting of SEQ ID NO: 2 to SEQ ID NO: 7
are detected.
Further, for example,
(i) the miRNAs according to SEQ ID NO: 2 and SEQ ID NO: 3,
(ii) the miRNAs according to SEQ ID NO: 2, SEQ ID NO: 3, and SEQ ID NO: 4,
(iii) the miRNAs according to SEQ ID NO: 2, SEQ ID NO: 3, SEQ ID NO: 4, and SEQ ID NO: 5,
(iv) the miRNAs according to SEQ ID NO: 2, SEQ ID NO: 3, SEQ ID NO: 4, SEQ ID NO: 5, and SEQ ID NO: 6,
(v) the miRNAs according to SEQ ID NO: 2, SEQ ID NO: 3, SEQ ID NO: 4, SEQ ID NO: 5, SEQ ID NO: 6, and SEQ ID NO: 7, and
(vi) the miRNAs according to SEQ ID NO: 2, SEQ ID NO: 3, SEQ ID NO: 4, SEQ ID NO: 5, SEQ ID NO: 6, SEQ ID NO: 7, and SEQ ID NO: 8
are detected.
More specifically, the at least one miRNA is selected from the group consisting of SEQ ID NO: 3 to SEQ ID NO: 5, and SEQ ID NO: 8.
For example,
(i) the miRNAs according to SEQ ID NO: 3 and SEQ ID NO: 4,
(ii) the miRNAs according to SEQ ID NO: 3 and SEQ ID NO: 5,
(iii) the miRNAs according to SEQ ID NO: 3 and SEQ ID NO: 8,
(iv) the miRNAs according to SEQ ID NO: 4 and SEQ ID NO: 5,
(v) the miRNAs according to SEQ ID NO: 4 and SEQ ID NO: 8,
(vi) the miRNAs according to SEQ ID NO: 5 and SEQ ID NO: 8,
(vii) the miRNAs according to SEQ ID NO: 3, SEQ ID NO: 4, and SEQ ID NO: 5,
(viii) the miRNAs according to SEQ ID NO: 3, SEQ ID NO: 4, and SEQ ID NO: 8,
(ix) the miRNAs according to SEQ ID NO: 3, SEQ ID NO: 5, and SEQ ID NO: 8,
(x) the miRNAs according to SEQ ID NO: 4, SEQ ID NO: 5, and SEQ ID NO: 8, and
(xi) the miRNAs according to SEQ ID NO: 3, SEQ ID NO: 4, SEQ ID NO: 5, and SEQ ID NO: 8
are detected.

It is more preferred that the at least one miRNA are one or more sets of miRNAs, wherein the one or more sets of miRNAs are listed in Figure 8.

All combinations of 2, 3, 4, 5, 6, or 7 miRNAs selected from the group consisting of SEQ ID NO: 2 to SEQ ID NO: 8 as well as the single miRNAs which can be detected are comprised in FIGURE 11.

The at least one miRNA is detected by determining the level, preferably the expression level, of said at least one miRNA. As mentioned above, the determination of the level of the at least one miRNA may be carried out by any convenient means for determining the level of a nucleotide sequence such as miRNA. For this purpose, qualitative, semi-quantitative and quantitative detection methods can be used. Quantitative detection methods are preferred. A variety of techniques are well known to the person skilled in the art. It is preferred that the level of the at least one miRNA representative for triple-negative breast cancer in a blood cellular fraction from a patient is determined by nucleic acid hybridization, nucleic acid amplification, polymerase extension, sequencing, mass spectroscopy, or any combination thereof. Regarding further explanations to these specific techniques, it is referred to the first aspect of the present invention.

The at least one polynucleotide is useful for conducting the method according to the first aspect of the present invention.

In a third aspect, the present invention relates to the use of at least one miRNA (e.g. 1, 2, 3, 4, 5, 6, 7, 8, 9, 10, 11, 12, 13, 14, 15, 16, 17, 18, 19, 20, 21, 22, or 23 miRNA(s)) isolated from a blood cellular fraction (particularly comprising or consisting of erythrocytes, leukocytes, and thrombocytes) from a patient for diagnosing triple-negative breast cancer (TNBC), wherein the at least one miRNA (e.g. 1, 2, 3, 4, 5, 6, 7, 8, 9, 10, 11, 12, 13, 14, 15, 16, 17, 18, 19, 20, 21, 22, or 23 miRNA(s)) is selected from the group consisting of SEQ ID NO: 1 to SEQ ID NO: 23, and a sequence having at least 80%, more preferably at least 85%, even more preferably at least 90%, and most preferably at least 95%, i.e. 80, 81, 82, 83, 84, 85, 86, 87, 88, 89, 90, 91, 92, 93, 94, 95, 96, 97, 98, or 99%, sequence identity thereto.

It is further preferred that the blood cellular fraction comprises erythrocytes, leukocytes, and thrombocytes. As mentioned above, the blood cellular fraction is produced from whole blood by removing the extracellular fraction (serum and/or plasma). In other words, the blood cellular fraction is depleted of the extracellular blood components, such as serum and/or plasma.

As mentioned above, at least one miRNA isolated from a blood cellular fraction from a patient is used for diagnosing triple-negative breast cancer.
It is preferred that the at least one miRNA is selected from the group consisting of SEQ ID NO: 1 to SEQ ID NO: 8.
For example, the miRNA according to SEQ ID NO: 1, SEQ ID NO: 2, SEQ ID NO: 3, SEQ ID NO: 4, SEQ ID NO: 5, SEQ ID NO: 6, SEQ ID NO: 7, or SEQ ID NO: 8 is used.
More specifically, the at least one miRNA is selected from the group consisting of SEQ ID NO: 1 to SEQ ID NO: 6 and SEQ ID NO: 8, or the at least one miRNA is selected from the group consisting of SEQ ID NO: 2 to SEQ ID NO: 8.
For example,
(i) the miRNA according to SEQ ID NO: 2 and at least one further miRNA selected from the group consisting of SEQ ID NO: 3 to SEQ ID NO: 8,
(ii) the miRNA according to SEQ ID NO: 3 and at least one further miRNA selected from the group consisting of SEQ ID NO: 2 and SEQ ID NO: 4 to SEQ ID NO: 8,
(iii) the miRNA according to SEQ ID NO: 4 and at least one further miRNA selected from the group consisting of SEQ ID NO: 2, SEQ ID NO: 3, and SEQ ID NO: 5 to SEQ ID NO: 8,
(iv) the miRNA according to SEQ ID NO: 5 and at least one further miRNA selected from the group consisting of SEQ ID NO: 2 to SEQ ID NO: 4 and SEQ ID NO: 6 to SEQ ID NO: 8,
(v) the miRNA according to SEQ ID NO: 6 and at least one further miRNA selected from the group consisting of SEQ ID NO: 2 to SEQ ID NO: 5, SEQ ID NO: 7, and SEQ ID NO: 8,
(vi) the miRNA according to SEQ ID NO: 7 and at least one further miRNA selected from the group consisting of SEQ ID NO: 2 to SEQ ID NO: 6 and SEQ ID NO: 8, and
(vii) the miRNA according to SEQ ID NO: 8 and at least one further miRNA selected from the group consisting of SEQ ID NO: 2 to SEQ ID NO: 7
are used.
Further, for example,
(i) the miRNAs according to SEQ ID NO: 2 and SEQ ID NO: 3,
(ii) the miRNAs according to SEQ ID NO: 2, SEQ ID NO: 3, and SEQ ID NO: 4,
(iii) the miRNAs according to SEQ ID NO: 2, SEQ ID NO: 3, SEQ ID NO: 4, and SEQ ID NO: 5,
(iv) the miRNAs according to SEQ ID NO: 2, SEQ ID NO: 3, SEQ ID NO: 4, SEQ ID NO: 5, and SEQ ID NO: 6,
(v) the miRNAs according to SEQ ID NO: 2, SEQ ID NO: 3, SEQ ID NO: 4, SEQ ID NO: 5, SEQ ID NO: 6, and SEQ ID NO: 7, and
(vi) the miRNAs according to SEQ ID NO: 2, SEQ ID NO: 3, SEQ ID NO: 4, SEQ ID NO: 5, SEQ ID NO: 6, SEQ ID NO: 7, and SEQ ID NO: 8
are used.
More specifically, the at least one miRNA is selected from the group consisting of SEQ ID NO: 3 to SEQ ID NO: 5, and SEQ ID NO: 8.
For example,
(i) the miRNAs according to SEQ ID NO: 3 and SEQ ID NO: 4,
(ii) the miRNAs according to SEQ ID NO: 3 and SEQ ID NO: 5,
(iii) the miRNAs according to SEQ ID NO: 3 and SEQ ID NO: 8,
(iv) the miRNAs according to SEQ ID NO: 4 and SEQ ID NO: 5,
(v) the miRNAs according to SEQ ID NO: 4 and SEQ ID NO: 8,
(vi) the miRNAs according to SEQ ID NO: 5 and SEQ ID NO: 8,
(vii) the miRNAs according to SEQ ID NO: 3, SEQ ID NO: 4, and SEQ ID NO: 5,
(viii) the miRNAs according to SEQ ID NO: 3, SEQ ID NO: 4, and SEQ ID NO: 8,
(ix) the miRNAs according to SEQ ID NO: 3, SEQ ID NO: 5, and SEQ ID NO: 8,
(x) the miRNAs according to SEQ ID NO: 4, SEQ ID NO: 5, and SEQ ID NO: 8, and
(xi) the miRNAs according to SEQ ID NO: 3, SEQ ID NO: 4, SEQ ID NO: 5, and SEQ ID NO: 8
are used.

It is more preferred that the at least one miRNA are one or more sets of miRNAs, wherein the one or more sets of miRNAs are listed in Figure 8.

All combinations of 2, 3, 4, 5, 6, or 7 miRNAs selected from the group consisting of SEQ ID NO: 2 to SEQ ID NO: 8 as well as the single miRNAs which can be used are comprised in FIGURE 11.

In a fourth aspect, the present invention relates to the use of a kit for diagnosing triple-negative breast cancer (TNBC) in a patient comprising:
(i) means for determining the level of at least one miRNA (e.g. 1, 2, 3, 4, 5, 6, 7, 8, 9, 10, 11, 12, 13, 14, 15, 16, 17, 18, 19, 20, 21, 22, or 23 miRNA(s)) representative for triple-negative breast cancer in a blood cellular fraction (particularly comprising or consisting of erythrocytes, leukocytes, and thrombocytes) from the patient, and
(ii) optionally a tube for blood sample storage,
wherein the at least one miRNA (e.g. 1, 2, 3, 4, 5, 6, 7, 8, 9, 10, 11, 12, 13, 14, 15, 16, 17, 18, 19, 20, 21, 22, or 23 miRNA(s)) is selected from the group consisting of SEQ ID NO: 1 to SEQ ID NO: 23, and a sequence having at least 80%, more preferably at least 85%, even more preferably at least 90%, and most preferably at least 95%, i.e. 80, 81, 82, 83, 84, 85, 86, 87, 88, 89, 90, 91, 92, 93, 94, 95, 96, 97, 98, or 99%, sequence identity thereto.

As mentioned above, the kit comprises means for determining the level of at least one miRNA representative for triple-negative breast cancer in a blood cellular fraction from a patient.
It is preferred that the at least one miRNA is selected from the group consisting of SEQ ID NO: 1 to SEQ ID NO: 8, and a sequence having at least 80% sequence identity thereto.
For example, the level of the miRNA according to SEQ ID NO: 1, SEQ ID NO: 2, SEQ ID NO: 3, SEQ ID NO: 4, SEQ ID NO: 5, SEQ ID NO: 6, SEQ ID NO: 7, or SEQ ID NO: 8 is determined.
More specifically, the at least one miRNA is selected from the group consisting of SEQ ID NO: 1 to SEQ ID NO: 6, SEQ ID NO: 8, and a sequence having at least 80% sequence identity thereto, or the at least one miRNA is selected from the group consisting of SEQ ID NO: 2 to SEQ ID NO: 8, and a sequence having at least 80% sequence identity thereto.
For example,
(i) the level of the miRNA according to SEQ ID NO: 2 and the level of at least one further miRNA selected from the group consisting of SEQ ID NO: 3 to SEQ ID NO: 8,
(ii) the level of the miRNA according to SEQ ID NO: 3 and the level of at least one further miRNA selected from the group consisting of SEQ ID NO: 2 and SEQ ID NO: 4 to SEQ ID NO: 8,
(iii) the level of the miRNA according to SEQ ID NO: 4 and the level of at least one further miRNA selected from the group consisting of SEQ ID NO: 2, SEQ ID NO: 3, and SEQ ID NO: 5 to SEQ ID NO: 8,
(iv) the level of the miRNA according to SEQ ID NO: 5 and the level of at least one further miRNA selected from the group consisting of SEQ ID NO: 2 to SEQ ID NO: 4 and SEQ ID NO: 6 to SEQ ID NO: 8,
(v) the level of the miRNA according to SEQ ID NO: 6 and the level of at least one further miRNA selected from the group consisting of SEQ ID NO: 2 to SEQ ID NO: 5, SEQ ID NO: 7, and SEQ ID NO: 8,
(vi) the level of the miRNA according to SEQ ID NO: 7 and the level of at least one further miRNA selected from the group consisting of SEQ ID NO: 2 to SEQ ID NO: 6 and SEQ ID NO: 8, and
(vii) the level of the miRNA according to SEQ ID NO: 8 and the level of at least one further miRNA selected from the group consisting of SEQ ID NO: 2 to SEQ ID NO: 7
is determined.
Further, for example,
(i) the level of the miRNAs according to SEQ ID NO: 2 and SEQ ID NO: 3,
(ii) the level of the miRNAs according to SEQ ID NO: 2, SEQ ID NO: 3, and SEQ ID NO: 4,
(iii) the level of the miRNAs according to SEQ ID NO: 2, SEQ ID NO: 3, SEQ ID NO: 4, and SEQ ID NO: 5,
(iv) the level of the miRNAs according to SEQ ID NO: 2, SEQ ID NO: 3, SEQ ID NO: 4, SEQ ID NO: 5, and SEQ ID NO: 6,
(v) the level of the miRNAs according to SEQ ID NO: 2, SEQ ID NO: 3, SEQ ID NO: 4, SEQ ID NO: 5, SEQ ID NO: 6, and SEQ ID NO: 7, and
(vi) the level of the miRNAs according to SEQ ID NO: 2, SEQ ID NO: 3, SEQ ID NO: 4, SEQ ID NO: 5, SEQ ID NO: 6, SEQ ID NO: 7, and SEQ ID NO: 8
is determined.
More specifically, the at least one miRNA is selected from the group consisting of SEQ ID NO: 3 to SEQ ID NO: 5, SEQ ID NO: 8 and a sequence having at least 80% sequence identity thereto.
For example,
(i) the level of the miRNAs according to SEQ ID NO: 3 and SEQ ID NO: 4,
(ii) the level of the miRNAs according to SEQ ID NO: 3 and SEQ ID NO: 5,
(iii) the level of the miRNAs according to SEQ ID NO: 3 and SEQ ID NO: 8,
(iv) the level of the miRNAs according to SEQ ID NO: 4 and SEQ ID NO: 5,
(v) the level of the miRNAs according to SEQ ID NO: 4 and SEQ ID NO: 8,
(vi) the level of the miRNAs according to SEQ ID NO: 5 and SEQ ID NO: 8,
(vii) the level of the miRNAs according to SEQ ID NO: 3, SEQ ID NO: 4, and SEQ ID NO: 5,
(viii) the level of the miRNAs according to SEQ ID NO: 3, SEQ ID NO: 4, and SEQ ID NO: 8,
(ix) the level of the miRNAs according to SEQ ID NO: 3, SEQ ID NO: 5, and SEQ ID NO: 8,
(x) the level of the miRNAs according to SEQ ID NO: 4, SEQ ID NO: 5, and SEQ ID NO: 8, and
(xi) the level of the miRNAs according to SEQ ID NO: 3, SEQ ID NO: 4, SEQ ID NO: 5, and SEQ ID NO: 8
is determined.

It is more preferred that the at least one miRNA are one or more sets of miRNAs, wherein the one or more sets of miRNAs are listed in Figure 8.

All combinations of 2, 3, 4, 5, 6, or 7 miRNAs selected from the group consisting of SEQ ID NO: 2 to SEQ ID NO: 8 as well as the single miRNAs which level can be determined are comprised in FIGURE 11.

It is further preferred that the means for determining the level of the at least one miRNA representative for triple-negative breast cancer in a blood cellular fraction from a patient comprise at least one polynucleotide as defined in the second aspect of the present invention. Said means for determining the level of the at least one miRNA representative for triple-negative breast cancer in a blood cellular fraction from the patient may further comprise a set of at least two primer pairs. Said at least two primer pairs allow the determination of the level of the at least one miRNA representative for triple-negative breast cancer in a blood cellular fraction from the patient.
Said means for determining the level of the at least one miRNA representative for triple-negative breast cancer in a blood cellular fraction from the patient may also comprise a microarray/biochip, a RT-PCT system, a PCR-system, a flow cytometer, a bead-based multiplex system or a next generation sequencing system. The at least one polynucleotide may be part of the microarray/biochip or may be attached to the beads of the beads-based multiplex system.

As mentioned above, the kit optionally comprises a tube for blood sample storage. The tube for blood sample storage may be any tube which allows storage of a blood sample for a sufficient time by preventing blood degradation. In particular, the tube for blood sample storage may be a tube which protects the RNA-fraction against degradation. Such a tube may be a PAXgene blood RNA tube. The PAXgene blood RNA tube is a special blood collection tube that has been developed to analyze RNA expression in blood cells (intra-cellular RNA). It contains a proprietary reagent that stabilizes intra-cellular RNA (RNA present in blood cells, including miRNA). After blood collection, the blood is incubated in order to allow the proprietary reagent to stabilize the intracellular RNA. Afterwards, the blood collection tube is centrifuged, which results in the separation of the solid (cellular, blood cells) component of blood and the liquid (extra-cellular, plasma/serum) component of blood. While the liquid component is discarded, the pelleted solid component is isolated and used for downstream (miRNA-) analyses.

It is preferred that blood cellular fraction comprises erythrocytes, leukocytes, and thrombocytes. As mentioned above, the blood cellular fraction is produced from whole blood by removing the extracellular fraction (serum and/or plasma). In other words, the blood cellular fraction is depleted of the extracellular blood components, such as serum and/or plasma.

The kit may further comprise a data carrier. Said data carrier may be a non-electronical data carrier, e.g. a graphical data carrier such as an information leaflet, an information sheet, a bar code or an access code, or an electronical data carrier such as a floppy disk, a compact disk (CD), a digital versatile disk (DVD), a microchip or another semiconductor-based electronical data carrier. The access code may allow the access to a database, e.g. an internet database, a centralized, or a decentralized database. The access code may also allow access to an application software that causes a computer to perform tasks for computer users or a mobile app which is a software designed to run on smartphones and other mobile devices.
Said data carrier may further comprise a reference level of the level of the at least one miRNA determined herein. In case that the data carrier comprises an access code which allows the access to a database, said reference level may be deposited in this database.

The kit may be used for conducting the method according to the first aspect of the present invention. The data carrier may comprise information or instructions on how to carry out the method according to the first aspect of the present invention.

Said kit may also comprise materials desirable from a commercial and user standpoint including a buffer(s), a reagent(s) and/or a diluent(s) for determining the level mentioned above.

The present inventors analysed miRNA expression profiles of patients prior to therapeutic treatment, in particular chemotherapy, and after therapeutic treatment, in particular chemotherapy. They surprisingly identified miRNAs which allow to determine whether a patient will respond to a therapeutic treatment or not. In addition, the present inventors identified a specific sample type, namely a blood cellular fraction comprising erythrocytes, leukocytes, and thrombocytes, as a special source of miRNAs having a high diagnostic potential.

Thus, described herein but not encompassed by the present invention is a method for determining whether a patient will respond to a therapeutic treatment of breast cancer comprising the step of:
determining the level of at least one miRNA (e.g. 1 or 2 miRNA(s)) associated with breast cancer in a blood sample (e.g. a blood cellular fraction, particularly comprising or consisting of erythrocytes, leukocytes, and thrombocytes) from the patient.
Preferably, the at least one miRNA (e.g. 1 or 2 miRNA(s)) is selected from the group consisting of SEQ ID NO: 22, SEQ ID NO: 25, and a sequence having at least 80%, more preferably at least 85%, even more preferably at least 90%, and most preferably at least 95%, i.e. 80, 81, 82, 83, 84, 85, 86, 87, 88, 89, 90, 91, 92, 93, 94, 95, 96, 97, 98, or 99%, sequence identity thereto. Accordingly, it is preferred that the method for determining whether a patient will respond to a therapeutic treatment of breast cancer comprises the step of:
determining the level of at least one miRNA (e.g. 1, or 2 miRNA(s)) associated with breast cancer in a blood sample (e.g. a blood cellular fraction, particularly comprising erythrocytes, leukocytes, and thrombocytes) from the patient, wherein the at least one miRNA (e.g. 1 or 2 miRNA(s)) is selected from the group consisting of SEQ ID NO: 22, SEQ ID NO: 25, and a sequence having at least 80%, more preferably at least 85%, even more preferably at least 90%, and most preferably at least 95%, i.e. 80, 81, 82, 83, 84, 85, 86, 87, 88, 89, 90, 91, 92, 93, 94, 95, 96, 97, 98, or 99%, sequence identity thereto.

In one embodiment, the level of the at least one miRNA is compared to a reference level of said at least one miRNA. Thus, in one particular embodiment, the method for determining whether a patient will respond to a therapeutic treatment of breast cancer comprises the steps of:
(i) determining the level of at least one miRNA (e.g. 1 or 2 miRNA(s)) associated with breast cancer in a blood sample (e.g. a blood cellular fraction, particularly comprising erythrocytes, leukocytes, and thrombocytes) from the patient,
   wherein the at least one miRNA (e.g. 1 or 2 miRNA(s)) is selected from the group consisting of SEQ ID NO: 22, SEQ ID NO: 25, and a sequence having at least 80%, more preferably at least 85%, even more preferably at least 90%, and most preferably at least 95%, i.e. 80, 81, 82, 83, 84, 85, 86, 87, 88, 89, 90, 91, 92, 93, 94, 95, 96, 97, 98, or 99%, sequence identity thereto, and
(ii) comparing the level of the at least one miRNA to a reference level of said at least one miRNA.
The above comparison allows for the determining whether the patient will respond to a therapeutic treatment of breast cancer.

The reference level may be any level which allows to determine whether a patient will respond to a therapeutic treatment of breast cancer or not. It is preferred that the reference level is the level determined by measuring at least one reference blood sample from at least one healthy subject. Preferably said reference blood samples are at least two reference blood samples, more preferably at least 2 to 100 reference blood samples, even more preferably at least 10 to 500 reference blood samples, and most preferably at least 50 to 10.000 reference blood samples, e.g. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, 1.000, 2.000, 3.000, 4.000, 5.000, or 10.000 reference blood samples. Preferably, said healthy subjects are at least two healthy subjects, more preferably at least 2 to 100 healthy subjects, even more preferably at least 10 to 500 healthy subjects, and most preferably at least 50 to 10.000 healthy subjects, e.g. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, 1.000, 2.000, 3.000, 4.000, 5.000, or 10.000 healthy subjects. It is practicable to take one reference blood sample per subject for analysis. If additional reference blood samples are required, e.g. to determine the reference level in different reference blood samples, the same subject may be (re)tested. Said reference level may be an average reference level. It may be determined by measuring reference levels and calculating the "average" value (e.g. mean, median or modal value) thereof. It is preferred that the reference blood sample is from the same source (e.g. whole blood or a blood cellular fraction comprising erythrocytes, leukocytes, and thrombocytes) than the blood sample taken from the patient. It is further preferred that the reference level is obtained from a subject of the same gender (e.g. female or male) and/or of a similar age/phase of life (e.g. adults or elderly) than the patient to be tested.

As mentioned above, the level of the at least one miRNA is compared to a reference level of said at least one miRNA. Said reference level is the level determined by measuring a reference blood sample. For example, if the level of the miRNA according to SEQ ID NO: 22 is determined in a blood sample from a patient, it is compared to a reference level of the miRNA according to SEQ ID NO: 22 determined in a reference blood sample. Alternatively, if the level of the miRNA according to SEQ ID NO: 22 and the level of the miRNA according to SEQ ID NO: 25 is determined in a blood sample from a patient, both levels are compared to the respective reference levels, i.e. the level of the miRNA according to SEQ ID NO: 22 is compared to the reference level of the miRNA according to SEQ ID NO: 22 and the level of the miRNA according to SEQ ID NO: 25 is compared to the reference level of the miRNA according to SEQ ID NO: 25 determined in a reference blood sample.

It is further preferred that
the level of the miRNA according to SEQ ID NO: 22 is above the reference level determined by measuring at least one reference blood sample from at least one healthy subject, which indicates that the patient will respond to the therapeutic treatment of breast cancer, and/or
the level of the miRNA according to SEQ ID NO: 25 is below the reference level determined by measuring at least one reference blood sample from at least one healthy subject, which indicates that the patient will respond to the therapeutic treatment of breast cancer.
Preferably, the level of the at least one miRNA is at least 0.5 fold above/below the reference level, more preferably at least 1.0-fold above/below the reference level, even more preferably at least 1.5-fold above/below the reference level, most preferably at least 2.0-fold above/below the reference level.

Alternatively or additionally, it is preferred that the reference level is the level determined by measuring at least one reference blood sample from at least one subject known as non-responder to the therapeutic treatment of breast cancer. Preferably said reference blood samples are at least two reference blood samples, more preferably at least 2 to 100 reference blood samples, even more preferably at least 10 to 500 reference blood samples, and most preferably at least 50 to 10.000 reference blood samples, e.g. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, 1.000, 2.000, 3.000, 4.000, 5.000, or 10.000 reference blood samples. Preferably, said subjects known as non-responders to the therapeutic treatment of breast cancer are at least two subjects known as non-responders to the therapeutic treatment of breast cancer, more preferably at least 2 to 100 subjects known as non-responders to the therapeutic treatment of breast cancer, even more preferably at least 10 to 500 subjects known as non-responders to the therapeutic treatment of breast cancer, and most preferably at least 50 to 10.000 subjects known as non-responders to the therapeutic treatment of breast cancer, e.g. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, 1.000, 2.000, 3.000, 4.000, 5.000, or 10.000 subjects known as non-responders to the therapeutic treatment of breast cancer. It is practicable to take one reference blood sample per subject for analysis. If additional reference blood samples are required, e.g. to determine the reference level in different reference blood samples, the same subject may be (re)tested. Said reference level may be an average reference level. It may be determined by measuring reference levels and calculating the "average" value (e.g. mean, median or modal value) thereof. It is preferred that the reference blood sample is from the same source (e.g. whole blood or a blood cellular fraction comprising erythrocytes, leukocytes, and thrombocytes) than the blood sample taken from the patient. It is further preferred that the reference level is obtained from a subject of the same gender (e.g. female or male) and/or of a similar age/phase of life (e.g. adults or elderly) than the patient to be tested.

It is further preferred that
the level of the miRNA according to SEQ ID NO: 22 is above the reference level determined by measuring at least one reference blood sample from at least one subject known as non-responder to the therapeutic treatment,
which indicates that the patient will respond to the therapeutic treatment of breast cancer, and/or the level of the miRNA according to SEQ ID NO: 25 is below the reference level determined by measuring at least one reference blood sample from at least one subject known as non-responder to the therapeutic treatment,
which indicates that the patient will respond to the therapeutic treatment of breast cancer.
Preferably, the level of the at least one miRNA is at least 0.5 fold above/below the reference level determined by measuring at least one reference blood sample from at least one subject known as non-responder to the therapeutic treatment, more preferably at least 1.0-fold above/below the reference level determined by measuring at least one reference blood sample from at least one subject known as non-responder to the therapeutic treatment.
In particular, with respect to the miRNA according to SEQ ID NO: 22, it is preferred that the level is at least 0.5 fold above the reference level determined by measuring at least one reference blood sample from at least one subject known as non-responder to the therapeutic treatment. It is more preferred that the level is at least 1.0-fold above the reference level determined by measuring at least one reference blood sample from at least one subject known as non-responder to the therapeutic treatment.
With respect to the miRNA according to SEQ ID NO: 25, it is preferred that the level is at least 0.5 fold below the reference level determined by measuring at least one reference blood sample from at least one subject known as non-responder to the therapeutic treatment.

It is more preferred that
the level of the miRNA according to SEQ ID NO: 22 is above the reference level determined by measuring at least one reference blood sample from at least one healthy subject and the reference level determined by measuring at least one reference blood sample from at least one subject known as non-responder to the therapeutic treatment,
which indicates that the patient will respond to the therapeutic treatment of breast cancer, and/or the level of the miRNA according to SEQ ID NO: 25 is below the reference level determined by measuring at least one reference blood sample from at least one healthy subject and the reference level determined by measuring at least one reference blood sample from at least one subject known as non-responder to the therapeutic treatment,
which indicates that the patient will respond to the therapeutic treatment of breast cancer.

In an alternative embodiment, an algorithm or a mathematical function is applied to the level of the at least one miRNA. Thus, in an alternative embodiment, the method for determining whether a patient will respond to a therapeutic treatment of breast cancer comprises the steps of:
(i) determining the level of at least one miRNA (e.g. 1 or 2 miRNA(s)) associated with breast cancer in a blood sample (e.g. a blood cellular fraction, particularly comprising erythrocytes, leukocytes, and thrombocytes) from the patient,
   wherein the at least one miRNA (e.g. 1 or 2 miRNA(s)) is selected from the group consisting of SEQ ID NO: 22, SEQ ID NO: 25, and a sequence having at least 80%, more preferably at least 85%, even more preferably at least 90%, and most preferably at least 95%, i.e. 80, 81, 82, 83, 84, 85, 86, 87, 88, 89, 90, 91, 92, 93, 94, 95, 96, 97, 98, or 99%, sequence identity thereto, and
(ii) applying an algorithm or mathematical function to the level of said at least one miRNA. The above application allows to determine whether the patient will respond to a therapeutic treatment of breast cancer.

The algorithm or mathematical function may be any algorithm or mathematical function which allows to decide if the patient will respond to a therapeutic treatment of breast cancer or not. It is preferred that the algorithm or mathematical function is obtained from a reference level of at least one miRNA determined in at least one blood sample from at least one subject known as non-responder to the therapeutic treatment of breast cancer, and a reference level of at least one miRNA determined in at least one blood sample from at least one healthy subject.
Preferably, said reference blood samples are at least two reference blood samples, more preferably at least 2 to 100 reference blood samples, even more preferably at least 10 to 500 reference blood samples, and most preferably at least 50 to 10.000 reference blood samples, e.g. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, 1.000, 2.000, 3.000, 4.000, 5.000, or 10.000 reference blood samples. Preferably, said healthy subjects are at least two healthy subjects, more preferably at least 2 to 100 healthy subjects, even more preferably at least 10 to 500 healthy subjects, and most preferably at least 50 to 10.000 healthy subjects, e.g. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, 1.000, 2.000, 3.000, 4.000, 5.000, or 10.000 healthy subjects. Preferably, said subjects known as non-responders to the therapeutic treatment of breast cancer are at least two subjects known as non-responders to the therapeutic treatment of breast cancer, more preferably at least 2 to 100 subjects known as non-responders to the therapeutic treatment of breast cancer, even more preferably at least 10 to 500 subjects known as non-responders to the therapeutic treatment of breast cancer, and most preferably at least 50 to 10.000 subjects known as non-responders to the therapeutic treatment of breast cancer, e.g. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, 1.000, 2.000, 3.000, 4.000, 5.000, or 10.000 subjects known as non-responders to the therapeutic treatment of breast cancer. It is practicable to take one reference blood sample per subject for analysis. If additional reference blood samples are required, e.g. to determine the reference level in different reference blood samples, the same subject may be (re)tested. Said reference level may be an average reference level. It may be determined by measuring reference levels and calculating the "average" value (e.g. mean, median or modal value) thereof. It is preferred that the reference blood sample is from the same source (e.g. whole blood or a blood cellular fraction comprising erythrocytes, leukocytes, and thrombocytes) than the blood sample taken from the patient. It is further preferred that the reference level is obtained from a subject of the same gender (e.g. female or male) and/or of a similar age/phase of life (e.g. adults or elderly) than the patient to be tested or diagnosed.
It is more preferred that the algorithm or mathematical function is obtained using a machine learning approach.
It is even more preferred that the machine learning approach involves the following steps:
(i) inputting
   the reference level of at least one miRNA determined in at least one reference blood sample from at least one subject known as non-responder to the therapeutic treatment of breast cancer, and
   the reference level of at least one miRNA determined in at least one reference blood sample from at least one healthy subject, and
(ii) computing an algorithm or a mathematical function based on said reference levels that is suitable to distinguish between a patient that will respond to the therapeutic treatment of breast cancer or not.

In order to determine whether a patient will respond to a therapeutic treatment of breast cancer, the level of the miRNA according to SEQ ID NO: 22 or SEQ ID NO: 25 is measured, or the level of the miRNAs according to SEQ ID NO: 22 and SEQ ID NO: 25 are measured.

The determination of the level of the at least one miRNA may be carried out by any convenient means for determining the level of a nucleotide sequence such as miRNA. For this purpose, qualitative, semi-quantitative and quantitative detection methods can be used. Quantitative detection methods are preferred. A variety of techniques are well known to the person skilled in the art. It is preferred that the level of the at least one miRNA representative for breast cancer in a blood sample from a patient is determined by nucleic acid hybridization, nucleic acid amplification, polymerase extension, sequencing, mass spectroscopy, or any combination thereof. Regarding further information, it is referred to the first aspect of the present invention.

Preferably, the level of the at least one miRNA which is determined is the expression level. Accordingly, it is preferred that the method for determining whether a patient responds to a therapeutic treatment of breast cancer comprises the step of: determining the expression level of at least one miRNA associated with breast cancer in a blood sample from the patient.

It is also preferred that the blood sample is selected from the group consisting of whole blood and a blood cellular fraction. It is more preferred that the blood cellular fraction comprises erythrocytes, leukocytes, and thrombocytes. As mentioned above, the blood cellular fraction is produced from whole blood by removing the extracellular fraction (serum and/or plasma). In other words, the blood cellular fraction is depleted of the extracellular blood components, such as serum and/or plasma.

The therapeutic treatment may be selected from the group consisting of chemotherapy, surgery, and radiotherapy. Preferably, the therapeutic treatment is chemotherapy.

If the patient tested will be a non-responder to the therapeutic treatment of breast cancer, the therapy form may be changed. For example, instead of chemotherapy, surgery, and radiotherapy, a drug therapy may be used.

Preferably, breast cancer is triple-negative breast cancer (TNBC).

Described herein but not encompassed by the present invention is the use of at least one polynucleotide (e.g. 1 or 2 polynucleotide(s)) for detecting at least one miRNA (e.g. 1 or 2 miRNA(s)) for determining whether a patient will respond to a therapeutic treatment of breast cancer in a blood sample (e.g. a blood cellular fraction, particularly comprising or consisting of erythrocytes, leukocytes, and thrombocytes) from the patient.
Preferably, the at least one miRNA (e.g. 1 or 2 miRNA(s)) is selected from the group consisting of SEQ ID NO: 22 and SEQ ID NO: 25. Accordingly, it is preferred that at least one polynucleotide (e.g. 1 or 2 polynucleotide(s)) is used for detecting at least one miRNA (e.g. 1 or 2 miRNA (s)) for determining whether a patient will respond to a therapeutic treatment of breast cancer in a blood sample (e.g. a blood cellular fraction, particularly comprising erythrocytes, leukocytes, and thrombocytes) from the patient, wherein the at least one miRNA (e.g. 1 or 2 miRNA(s)) is selected from the group consisting of SEQ ID NO: 22 and SEQ ID NO: 25.

It is further preferred that
(i) the at least one polynucleotide is complementary to the at least one miRNA mentioned above, or
(ii) the at least one polynucleotide has at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity to the polynucleotide according to (i).
It is particularly preferred that the polynucleotide as defined in (ii) has at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity over a continuous stretch of at least 10, 11, 12, 13, 14, 15, 16, 17, 18, 19, 20, 21, 22, 23, 24, 25, or more nucleotides, preferably over the whole length, to the polynucleotide according to (i).

It is also preferred that the blood sample is selected from the group consisting of whole blood and a blood cellular fraction. It is more preferred that the blood cellular fraction comprises erythrocytes, leukocytes, and thrombocytes. As mentioned above, the blood cellular fraction is produced from whole blood by removing the extracellular fraction (serum and/or plasma). In other words, the blood cellular fraction is depleted of the extracellular blood components, such as serum and/or plasma.

As mentioned above, at least one polynucleotide is used for detecting at least one miRNA for determining whether a patient responds to a therapeutic treatment of breast cancer in a blood sample from the patient.
It is preferred that the at least one miRNA is the miRNA according to SEQ ID NO: 22 or SEQ ID NO: 25. It is more preferred that the at least one miRNA are the miRNAs according to SEQ ID NO: 22 and SEQ ID NO: 25.

The at least one polynucleotide is useful for conducting the method described above.

The therapeutic treatment may be selected from the group consisting of chemotherapy, surgery, and radiotherapy. Preferably, the therapeutic treatment is chemotherapy.

Preferably, breast cancer is triple-negative breast cancer (TNBC).

Described herein but not encompassed by the present invention is the use of at least one miRNA (e.g. 1 or 2 miRNA(s)) isolated from a blood sample (e.g. a blood cellular fraction, particularly comprising or consisting of erythrocytes, leukocytes, and thrombocytes) from a patient for determining whether the patient will respond to a therapeutic treatment of breast cancer.

Preferably, the at least one miRNA (e.g. 1 or 2 miRNA(s)) is selected from the group consisting of SEQ ID NO: 22, SEQ ID NO: 25, and a sequence having at least 80%, more preferably at least 85%, even more preferably at least 90%, and most preferably at least 95%, i.e. 80, 81, 82, 83, 84, 85, 86, 87, 88, 89, 90, 91, 92, 93, 94, 95, 96, 97, 98, or 99%, sequence identity thereto. Accordingly, it is preferred that at least one miRNA (e.g. 1 or 2 miRNA (s)) isolated from a blood sample (e.g. a blood cellular fraction, particularly comprising erythrocytes, leukocytes, and thrombocytes) from a patient is used for determining whether the patient will respond to a therapeutic treatment of breast cancer, wherein the at least one miRNA (e.g. 1 or 2 miRNA(s)) is selected from the group consisting of SEQ ID NO: 22, SEQ ID NO: 25, and a sequence having at least 80%, more preferably at least 85%, even more preferably at least 90%, and most preferably at least 95%, i.e. 80, 81, 82, 83, 84, 85, 86, 87, 88, 89, 90, 91, 92, 93, 94, 95, 96, 97, 98, or 99%, sequence identity thereto.

It is preferred that the blood sample is selected from the group consisting of whole blood and a blood cellular fraction. It is more preferred that the blood cellular fraction comprises erythrocytes, leukocytes, and thrombocytes. As mentioned above, the blood cellular fraction is produced from whole blood by removing the extracellular fraction (serum and/or plasma). In other words, the blood cellular fraction is depleted of the extracellular blood components, such as serum and/or plasma.

As mentioned above, at least one miRNA isolated from a blood sample from a patient is used for determining whether the patient will respond to a therapeutic treatment of breast cancer. It is preferred that the at least one miRNA is the miRNA according to SEQ ID NO: 22 or SEQ ID NO: 25. It is more preferred that the at least one miRNA are the miRNAs according to SEQ ID NO: 22 and SEQ ID NO: 25.

The therapeutic treatment may be selected from the group consisting of chemotherapy, surgery, and radiotherapy. Preferably, the therapeutic treatment is chemotherapy.

Preferably, breast cancer is triple-negative breast cancer (TNBC).

Described herein but not encompassed by the present invention is a kit for determining whether a patient will respond to a therapeutic treatment of breast cancer comprising:
(i) means for determining the level of at least one miRNA (e.g. 1 or 2 miRNA(s)) associated with breast cancer in a blood sample (e.g. a blood cellular fraction, particularly comprising or consisting of erythrocytes, leukocytes, and thrombocytes) from the patient, and
(ii) optionally a tube for blood sample storage.

Preferably, the at least one miRNA (e.g. 1 or 2 miRNA(s)) is selected from the group consisting of SEQ ID NO: 22 and SEQ ID NO: 25 and a sequence having at least 80%, more preferably at least 85%, even more preferably at least 90%, and most preferably at least 95%, i.e. 80, 81, 82, 83, 84, 85, 86, 87, 88, 89, 90, 91, 92, 93, 94, 95, 96, 97, 98, or 99%, sequence identity thereto. Accordingly, it is preferred that the kit for determining whether a patient will respond to a therapeutic treatment of breast cancer comprises:
(i) means for determining the level of at least one miRNA (e.g. 1 or 2 miRNA(s)) associated with breast cancer in a blood sample (e.g. a blood cellular fraction, particularly comprising erythrocytes, leukocytes, and thrombocytes) from the patient, and
(ii) optionally a tube for blood sample storage,
wherein the at least one miRNA (e.g. 1 or 2 miRNA(s)) is selected from the group consisting of SEQ ID NO: 22, SEQ ID NO: 25, and a sequence having at least 80%, more preferably at least 85%, even more preferably at least 90%, and most preferably at least 95%, i.e. 80, 81, 82, 83, 84, 85, 86, 87, 88, 89, 90, 91, 92, 93, 94, 95, 96, 97, 98, or 99%, sequence identity thereto.

As mentioned above, the kit comprises means for determining the level of at least one miRNA associated with breast cancer in a blood sample from a patient. It is preferred that the at least one miRNA is the miRNA according to SEQ ID NO: 22 or SEQ ID NO: 25. It is more preferred that the at least one miRNA are the miRNAs according to SEQ ID NO: 22 and SEQ ID NO: 25.

It is further preferred that the means for determining the level of the at least one miRNA associated with breast cancer in a blood sample from a patient comprise at least one polynucleotide as defined above.
Said means for determining the level of the at least one miRNA associated with breast cancer in a blood sample from the patient may further comprise a set of at least two primer pairs. Said at least two primer pairs allow the determination of the level of the at least one miRNA associated with breast cancer in a blood sample from a patient.
Said means for determining the level of the at least one miRNA associated with breast cancer in a blood sample from the patient may also comprise a microarray/biochip, a RT-PCT system, a PCR-system, a flow cytometer, a bead-based multiplex system or a next generation sequencing system. The at least one polynucleotide may be part of the microarray/biochip or may be attached to the beads of the beads-based multiplex system.

As mentioned above, the kit optionally comprises a tube for blood sample storage. The tube for blood sample storage may be any tube which allows storage of a blood sample for a sufficient time by preventing blood degradation. In particular, the tube for blood sample storage may be a tube which protects the RNA-fraction against degradation. Such a tube may be a PAXgene blood RNA tube. The PAXgene blood RNA tube is a special blood collection tube that has been developed to analyze RNA expression in blood cells. It contains a proprietary reagent that stabilizes intra-cellular RNA (RNA present in blood cells, including miRNA). After blood collection, the blood is incubated in order to allow the proprietary reagent to stabilize the intracellular RNA. Afterwards, the blood collection tube is centrifuged, which results in the separation of the solid (cellular, blood cells) component of blood and the liquid (extra-cellular, plasma/serum) component of blood. While the liquid component is discarded, the pelleted solid component is isolated and used for downstream (miRNA-) analyses. Alternatively, the tube for blood sample storage may be an EDTA tube, a heparin tube, a citrate tube, or a Tempus tube.

It is also preferred that the blood sample is selected from the group consisting of whole blood and a blood cellular fraction. It is more preferred that the blood cellular fraction comprises erythrocytes, leukocytes, and thrombocytes. As mentioned above, the blood cellular fraction is produced from whole blood by removing the extracellular fraction (serum and/or plasma). In other words, the blood cellular fraction is depleted of the extracellular blood components, such as serum and/or plasma.

The kit may further comprise a data carrier. Said data carrier may be a non-electronical data carrier, e.g. a graphical data carrier such as an information leaflet, an information sheet, a bar code or an access code, or an electronical data carrier such as a floppy disk, a compact disk (CD), a digital versatile disk (DVD), a microchip or another semiconductor-based electronical data carrier. The access code may allow the access to a database, e.g. an internet database, a centralized, or a decentralized database. The access code may also allow access to an application software that causes a computer to perform tasks for computer users or a mobile app which is a software designed to run on smartphones and other mobile devices.
Said data carrier may further comprise a reference level of the level of the at least one miRNA determined herein. In case that the data carrier comprises an access code which allows the access to a database, said reference level may be deposited in this database.

The kit may be used for conducting the method described above. The data carrier may comprise information or instructions on how to carry out the method described above.

Said kit may also comprise materials desirable from a commercial and user standpoint including a buffer(s), a reagent(s) and/or a diluent(s) for determining the level mentioned above.

Preferably, breast cancer is triple-negative breast cancer (TNBC).

Various modifications and variations of the invention will be apparent to those skilled in the art without departing from the scope of invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art in the relevant fields are intended to be covered by the present invention.

### BRIEF DESCRIPTION OF THE FIGURES

The following Figures are merely illustrative of the present invention and should not be construed to limit the scope of the invention as indicated by the appended claims in any way.
**Figure 1****:** MiRNAs for diagnosis of breast cancer (Differentiate between individuals suffering from breast cancer and individuals not suffering from breast cancer; with breast cancer = TNBC). Experimental data obtained for analysis of miRNAs according to SEQ ID NO: 1 to SEQ ID NO: 23. Experimental details: SEQ ID NO: sequence identification number, miRNA: identifier of the miRNA according to miRBase, median group 1: median intensity obtained from microarray analysis for healthy controls, median group 2: median intensity obtained from microarray analysis for individuals with breast cancer, fold change: ratio of median group 2/median group 1, Wilcoxon Mann Whitney Test adjusted p-Value: p-value obtained when applying Wilcoxon Mann Whitney with adjusted p-value in order to reduce false discovery rate by Benjamini-Hochberg adjustment ; ttest adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment.
**Figure 2****:** MiRNAs for diagnosis of breast cancer (differentiate between breast cancer individuals that will respond (positively) or will not respond (positively) to chemotherapy; with breast cancer = TNBC). Experimental data obtained for analysis of miRNAs according to SEQ ID NO: 22 and SEQ ID NO: 25. Experimental details: SEQ ID NO: sequence identification number, miRNA: identifier of the miRNA according to miRBase, median group 1 (Resp): median intensity obtained from microarray analysis for for breast cancer individuals that responded to chemotherapy, median group 2 (non-Resp): median intensity obtained from microarray analysis for breast cancer individuals that not responded to chemotherapy, fold change (non-Resp/Resp): ratio of median group 2 (non-Resp)/median group 1(Resp), Wilcoxon Mann Whitney Test adjusted p-Value: p-value obtained when applying Wilcoxon Mann Whitney with adjusted p-value in order to reduce false discovery rate by Benjamini-Hochberg adjustment ; ttest_adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment.

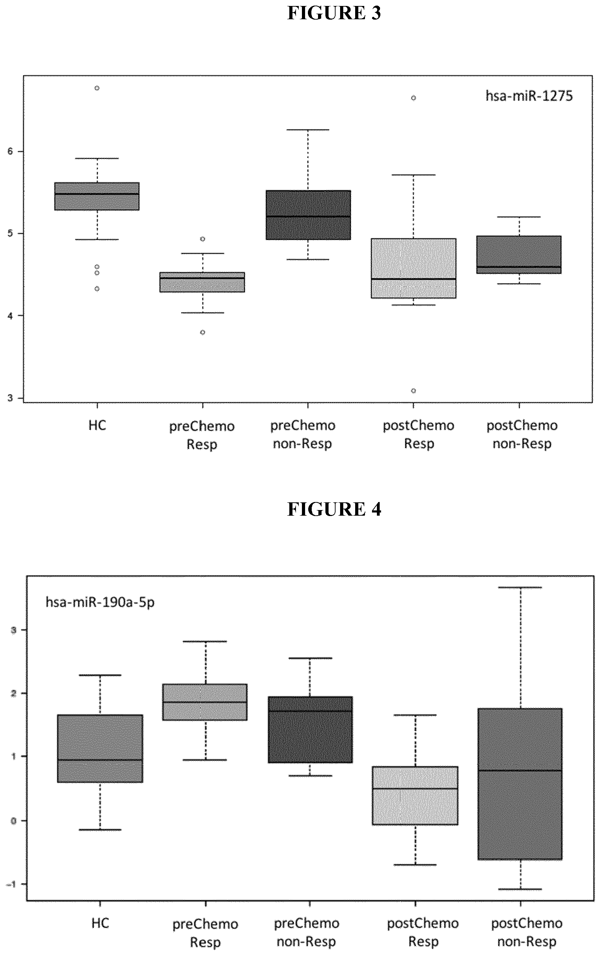

**Figure 3****:** hsa-miR-1275 (SEQ ID NO: 25) for diagnosis of breast cancer (TNBC). Depicted are the normalized expression levels (y-axis) in different individual groups : HC ( healthy control, not suffering from breast cancer), preChemo Resp (breast cancer individuals before chemotherapy, that lateron responded to chemotherapy), preChemo non-Resp (breast cancer individuals before chemotherapy, that lateron not responded to chemotherapy), postChemo Resp (breast cancer individuals after chemotherapy, that responded to chemotherapy), postChemo non-Resp (breast cancer individuals after chemotherapy, that not responded to chemotherapy).
**Figure 4****:** hsa-miR-190a-5p (SEQ ID NO: 22) for diagnosis of breast cancer (TNBC). Depicted are the normalized expression levels (y-axis) in different individual groups : HC ( healthy control, not suffering from breast cancer), preChemo Resp (breast cancer individuals before chemotherapy, that lateron responded to chemotherapy), preChemo non-Resp (breast cancer individuals before chemotherapy, that lateron not responded to chemotherapy), postChemo Resp (breast cancer individuals after chemotherapy, that responded to chemotherapy), postChemo non-Resp (breast cancer individuals after chemotherapy, that not responded to chemotherapy).

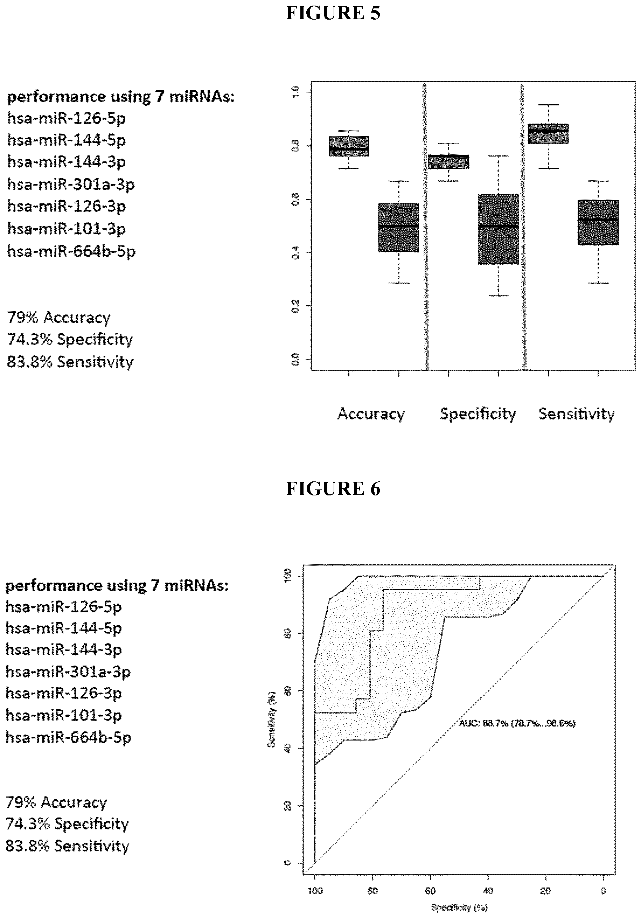

**Figure 5****:** MiRNAs for diagnosis of breast cancer (Differentiate between individuals suffering from breast cancer and individuals not suffering from breast cancer; with breast cancer = TNBC). Depicted is classification performance (accuracy, specificity, sensitivity) of a combination of 7 miRNAs, including hsa-miR-126-5p, hsa-miR-144-5p, hsa-miR-144-3p, hsa-miR-301a-3p, hsa-miR-126-3p, hsa-miR-101-3p, hsa-miR-664-5p. Herein, in each section (accuracy, specificity, sensitivity) the box-whisker plots on the left represent the classification performance of said combination of 7 miRNAs, while the box-whisker plots on the right represent the classification performance of a random selection of miRNAs. Herein, accuracy = 79 %, specificity = 74.3 %, sensitivity = 83.8 %.
**Figure 6****:** MiRNAs for diagnosis of breast cancer (Differentiate between individuals suffering from breast cancer and individuals not suffering from breast cancer; with breast cancer = TNBC). Depicted is the performance of a combination of 7 miRNAs in terms of AUC (88.7%); herein the combination of 7 miRNAs include hsa-miR-126-5p, hsa-miR-144-5p, hsa-miR-144-3p, hsa-miR-301a-3p, hsa-miR-126-3p, hsa-miR-101-3p, hsa-miR-664-5p. Herein, accuracy = 79 %, specificity = 74.3 %, sensitivity = 83.8 %.

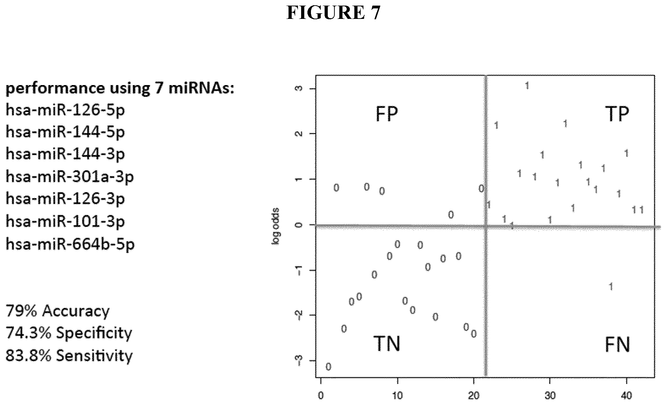

**Figure 7****:** MiRNAs for diagnosis of breast cancer (Differentiate between individuals suffering from breast cancer and individuals not suffering from breast cancer; with breast cancer = TNBC). Depicted is the performance of a combination of 7 miRNAs in terms of assignment of individual breast patients to FP=False Positive, TN = True Negative (Healthy Control), TP = True Positive (Breast Cancer) and FN = False Negative; herein the combination of 7 miRNAs include hsa-miR-126-5p, hsa-miR-144-5p, hsa-miR-144-3p, hsa-miR-301a-3p, hsa-miR-126-3p, hsa-miR-101-3p, hsa-miR-664-5p. Herein, accuracy = 79 %, specificity = 74.3 %, sensitivity = 83.8 %. Herein, accuracy = 79 %, specificity = 74.3 %, sensitivity = 83.8 %.
**Figure 8****:** Differentiate between individuals suffering from breast cancer and individuals not suffering from breast cancer (with breast cancer = TNBC). Depicted are the performances in respect to accuracy, specificity and sensitivity, as well as the AUC-value that are obtained when combining selected combinations of preferred miRNAs from the group consisting of SEQ ID NO: 1 to SEQ ID NO: 23. Herein, listed are combinations of 2 miRNAs with accuracy ≥ 70% and combinations of 3 miRNAs with accuracy ≥ 75%.

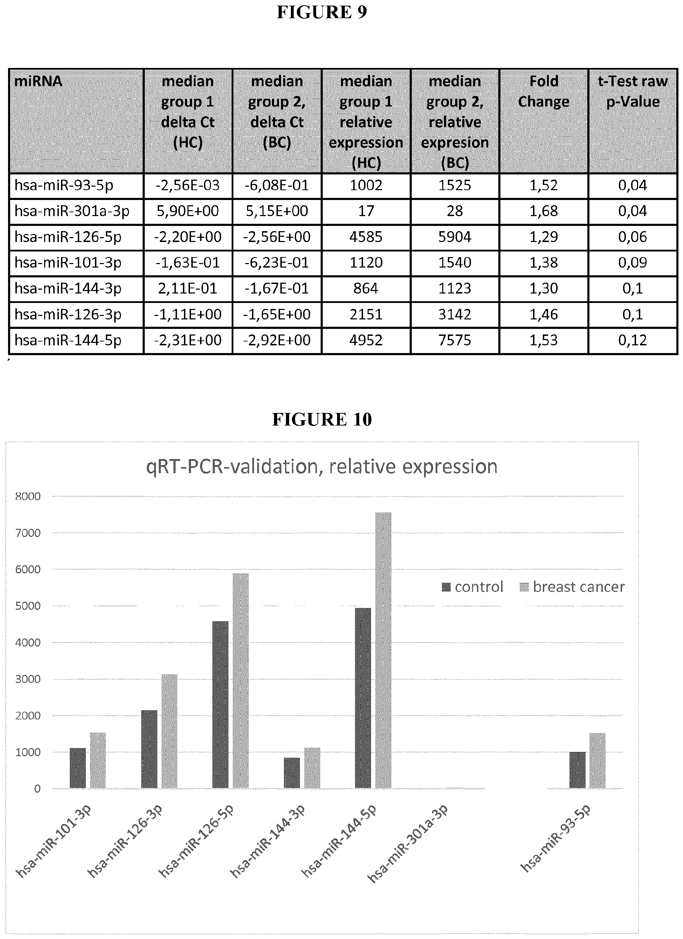

**Figure 9****:** qRT-PCR validation employing 7 miRNAs (hsa-miR-93-5p, hsa-miR-301a-3p, hsa-miR-126-5p, hsa-miR-101-3p, hsa-miR-144-3p, hsa-miR-126-3p, hsa-miR-144-5p) for diagnosis of breast cancer (Differentiate between individuals suffering from breast cancer (TNBC) and individuals not suffering from breast cancer).
   Experimental details: median group 1 delta Ct (HC)=median Ct-value from healthy controls normalized to reference RNU48; median group 2, delta Ct (BC) =median Ct-value from breast cancer subjects (TNBC) normalized to reference RNU48; median group 1 relative expression (HC) = median expression of healthy controls; median group 2, relative expression (BC) =median expression of breast cancer subjects (TNBC), Fold Change = median group 2 relative expression (HC) divided by median group 1, relative expression (BC); t-Test raw p-Value = p-value obtained when applying t-test
**Figure 10****:** graphical representation of qRT-PCR validation employing 7 miRNAs (hsa-miR-93-5p, hsa-miR-301a-3p, hsa-miR-126-5p, hsa-miR-101-3p, hsa-miR-144-3p, hsa-miR-126-3p, hsa-miR-144-5p) for diagnosis of breast cancer (Differentiate between individuals suffering from breast cancer (TNBC) and individuals not suffering from breast cancer).
**Figure 11****:** Summary of all combinations of 2, 3, 4, 5, 6, or 7 miRNAs selected from the group consisting of SEQ ID NO: 2 to SEQ ID NO: 8 as well as the single miRNAs which level can be determined.

### EXAMPLES

The examples given below are for illustrative purposes only and do not limit the invention described above in any way.

### 1. Materials and methods

### 1.1 Patient samples

Blood was collected from subjects suffering from 21 breast cancer (in particular triple-negative breast cancer subjects, TNBC) and 21 subjects not suffering from breast cancer (healthy controls). For each of the breast cancer subject 2 samples were collected, namely one sample prior chemotherapy and one sample after being subjected to chemotherapy.

### 1.2 Blood sample collection using PaxGene Blood RNA tubes

For each blood donor 2.5 ml of whole blood was collected by venous puncture into a PAXgene Blood RNA Tube (PreAnalytix, Hombrechticon, Switzerland). The blood cells were derived/obtained from processing the whole blood samples by centrifugation. Herein, the blood cells from the whole blood collected in said blood collection tubes were spun down by 10 min, 5000xg centrifugation. The blood cell pellet (the cellular blood fraction comprising red blood cells, white blood cells and platelets) was harvested for further processing, while the supernatant (including the extra-cellular blood fraction) was discarded. Total RNA, including the small RNA fraction (including the miRNA-fraction), was extracted from the harvested blood cells using the miRNeasy Mini Kit (Qiagen GmbH, Hilden, Germany).

### 1.3 Extraction of total RNA (incl. miRNA-fraction) from PAXgene Blood RNA tubes

The isolation of total RNA, including the small RNA fraction (including the miRNA-fraction) was performed by use of the miRNeasy Mini Kit (Qiagen GmbH, Hilden, Germany). Herein, the blood cell pellet (obtained as outlined above) was thoroughly resuspended in 700 µl QIAzol lysis reagent by pipetting up and down and immediately the suspension was transferred to a new 1,5 ml Eppendorf tube. Then 140 µl chloroform were added, vortexed thoroughly and incubated for 2-3 min at room temperature, followed by centrifugation at 12,000 g for 15 min at 4°C. Afterwards, the upper, aqueous phase was transferred to a new 2 ml tube with great care, without touching the other two phases. Then 1.5 volumes of 100% ethanol were added to the transferred aqueous phase and thoroughly mixing was done by pipetting. 700 µl of sample were then transferred into a column and centrifuged at 13,000 rpm for 15 sec at RT, discarding the flow-through. Afterwards 700 µl of Buffer RWT were added to each column, centrifuged again at 13,000 rpm for 15 sec at RT, discarding the flow-through. Then 500 µl Buffer RPE was added to the column and centrifuged at 13,000 rpm for 15 sec at RT, discarding the flow-through. Afterwards another 500 µl Buffer RPE was added to the column and centrifuged at 13,000 rpm for 2 min at RT, discarding the flow-through. Then the column was placed into a new 2 ml collection tube and centrifuged at 13,000 rpm for 1 min at RT to dry it. The column was transferred into a new 1.5 ml collection tube. For elution of the total RNA incl. microRNA 40 µl RNase-free water was pipetted onto the column and incubated for 1 min, centrifuged at 13.000 rpm at RT for 1 min. Then the eluate was put back onto the same column, incubated for 1 min at RT and centrifuged again for 1 min. The eluted total RNA, including the small RNA fraction (including the miRNA-fraction) was quantified using the NanoDrop 1000 and stored at -20°C before use in expression profiling experiments.

### 1.4 Quality Control of small RNA fraction

Quality control and quantification of extracted RNA was performed by using Agilent's Bioanalyzer (Agilent Technologies, Santa Clara, CA, USA) according to manufacturer's protocol employing both the Bioanalyzer Small and Nano Assay Kits The RNA was denatured for 2 min at 70°C, afterwards 1 µl was applied to the Bioanalyzer Chip for both Small and Nano Assay. Chips were vortexed at 2,400 rpm and run on Bioanalyzer Instrument within 5 min.

### 1.5 Microarray-based determination of microRNA expression profiles

Total RNA sample including microRNA-fraction was analyzed on Agilent human miRNA 8x60k microarray (release v21) according to manufacturer's protocol. After enzymatic Cy3-labeling of microRNA and 20 hours of array hybridization at 55°C in a rotating hybridization oven, the microarray slide was washed twice (non-stringent and stringent) and afterwards scanned with Agilent's SureScan Microarray Scanner. Resulting image data was evaluated using Agilent Feature Extraction software (v11). Raw data files (GeneView) generated by Feature Extraction software were imported in Excel for present call analysis.

### 1.6 Data Analysis, Statistics

To analyze the microarray miRNA measurements we used proprietary tools based on software implemented in the R programming environment for statistical computations. First, the data were normalized using variance-stabilizing normalization (VSN). Next, we determined for each miRNA in each patient whether the miRNA is expressed in the patient ("1") or not expressed in the patient ("0"). The binary flag has been set to 1, if the miRNA is at least three standard deviations above the local background and 0 otherwise.

To minimize the influence of miRNAs expressed close to the background only those markers with stable expression in at least three patients were considered while all other miRNAs were omitted.

For secondary statistical / bioinformatics analysis we applied Analysis of Variance after checking that data are approximately normally distributed using Shapiro-Wilk testing. Further, we carried out principal component analysis and bottom up hierarchical clustering using the Euclidean Distance measure. For assessing how differentially expressed miRNAs are between two cohorts we computed common statistical measures such as t-test, Wilcoxon Mann-Whitney test, fold changes and the Area Under The ROC curve. All p-values were adjusted for multiple testing using the Benjamini Hochberg Approach.

### REFERENCE

Variance stabilization applied to microarray data calibration and to the quantification of differential expression, Wolfgang Huber, Anja von Heydebreck, Holger Sueltmann, Annemarie Poustka, Martin Vingron; Bioinformatics (2002) 18 Suppl. 1 S96-S104.

## Claims

1. A method for diagnosing triple-negative breast cancer (TNBC) in a patient comprising the step of:
determining the level of at least one miRNA representative for triple-negative breast cancer in a blood cellular fraction from the patient,
wherein the at least one miRNA is selected from the group consisting of SEQ ID NO: 1 to SEQ ID NO: 23, and a sequence having at least 80% sequence identity thereto.

2. The method of claim 1, wherein the level of the at least one miRNA is compared to a reference level of said at least one miRNA.

3. The method of claim 2, wherein the reference level is the level determined by measuring at least one reference blood cellular fraction from at least one healthy subject.

4. The method of claim 3, wherein
the level of the at least one miRNA selected from the group consisting of SEQ ID NO: 1 to SEQ ID NO: 6, SEQ ID NO: 8 to SEQ ID NO: 14, and SEQ ID NO: 18 to SEQ ID NO: 23 above the reference level indicates that the patient has triple-negative breast cancer, and/or
the level of the at least one miRNA selected from the group consisting of SEQ ID NO: 7 and SEQ ID NO: 15 to SEQ ID NO: 17 below the reference level indicates that the patient has triple-negative breast cancer.

5. The method of any one of claims 1 to 4, wherein the blood cellular fraction comprises erythrocytes, leukocytes, and thrombocytes.

6. The method of any one of claims 1 to 5, wherein the at least one miRNA is selected from the group consisting of SEQ ID NO: 1 to SEQ ID NO: 8 and a sequence having at least 80% sequence identity thereto.

7. The method of any one of claims 1 to 6, wherein the at least one miRNA are one or more sets of miRNAs, wherein the one or more sets of miRNAs are listed in Figure 8.

8. Use of at least one polynucleotide for detecting at least one miRNA for diagnosing triple-negative breast cancer (TNBC) in a blood cellular fraction from a patient, wherein the at least one miRNA is selected from the group consisting of SEQ ID NO: 1 to SEQ ID NO: 23.

9. The use of claim 8, wherein
(i) the at least one polynucleotide is complementary to the at least one miRNA of claim 8, or
(ii) the at least one polynucleotide has at least 80% sequence identity to the polynucleotide according to (i).

10. The use of claims 8 or 9, wherein the blood cellular fraction comprises erythrocytes, leukocytes, and thrombocytes.

11. Use of at least one miRNA isolated from a blood cellular fraction from a patient for diagnosing triple-negative breast cancer (TNBC),
wherein the at least one miRNA is selected from the group consisting of SEQ ID NO: 1 to SEQ ID NO: 23, and a sequence having at least 80% sequence identity thereto.

12. The use of claim 11, wherein the blood cellular fraction comprises erythrocytes, leukocytes, and thrombocytes.

13. Use of a kit for diagnosing triple-negative breast cancer (TNBC) in a blood cellular fraction from a patient comprising
(i) means for determining the level of at least one miRNA representative for triple-negative breast cancer in a blood cellular fraction from a patient, and
(ii) optionally a tube for blood sample storage,
wherein the at least one miRNA is selected from the group consisting of SEQ ID NO: 1 to SEQ ID NO: 23, and a sequence having at least 80% sequence identity thereto.

14. Use of the kit of claim 13, wherein the means for determining the level of the at least one miRNA representative for triple-negative breast cancer in a blood cellular fraction from a patient comprise at least one polynucleotide as defined in any one of claims 8 to 10.

## Patentansprüche

1. Verfahren zur Diagnose von dreifach-negativem Brustkrebs (TNBC) bei einem Patienten, umfassend den Schritt:
Ermitteln des Levels von mindestens einer miRNA, die für den dreifach-negativen Brustkrebs repräsentativ ist, in einer Blutzellfraktion des Patienten, wobei die mindestens eine miRNA aus der Gruppe bestehend aus SEQ ID NO: 1 bis SEQ ID NO: 23 und einer Sequenz mit mindestens 80% Sequenzidentität hierzu ausgewählt ist.

2. Verfahren nach Anspruch 1, wobei das Level der mindestens einen miRNA mit einem Referenzlevel dieser mindestens einen miRNA verglichen wird.

3. Verfahren nach Anspruch 2, wobei das Referenzlevel das Level ist, das durch Messen mindestens einer Referenz-Blutzellfraktion von mindestens einem gesunden Subjekt erhalten wird.

4. Verfahren nach Anspruch 3, wobei
das Level der mindestens einen miRNA ausgewählt aus der Gruppe bestehend aus SEQ ID NO: 1 bis SEQ ID NO: 6, SEQ ID NO: 8 bis SEQ ID NO: 14 und SEQ ID NO: 18 bis SEQ ID NO: 23 oberhalb des Referenzlevels darauf hin deutet, dass der Patient dreifach-negative Brustkrebs hat, und/oder
das Level der mindestens einen miRNA ausgewählt aus der Gruppe bestehend aus SEQ ID NO: 7 und SEQ ID NO: 15 bis SEQ ID NO: 17 unterhalb des Referenzlevels darauf hindeutet, dass der Patient dreifach-negativen Brustkrebs hat.

5. Verfahren nach einem der Ansprüche 1 bis 4, wobei die Blutzellfraktion Erythrozyten, Leukozyten und Thrombozyten umfasst.

6. Verfahren nach einem der Ansprüche 1 bis 5, wobei die mindestens eine miRNA aus der Gruppe bestehend aus SEQ ID NO: 1 bis SEQ ID NO: 8 und einer Sequenz mit mindestens 80% Sequenzidentität hierzu ausgewählt ist.

7. Verfahren nach einem der Ansprüche 1 bis 6, wobei die mindestens eine miRNA ein Set ist oder mehrere Sets von miRNAs sind, wobei das eine Set oder die mehreren Sets von miRNAs in Figur 8 aufgeführt ist/sind.

8. Verwendung von mindestens einem Polynukleotid zum Nachweis von mindestens einer miRNA zur Diagnose von dreifach-negativem Brustkrebs (TNBC) in einer Blutzellfraktion eines Patienten,
wobei die mindestens eine miRNA aus der Gruppe bestehend aus SEQ ID NO: 1 bis SEQ ID NO: 23 ausgewählt ist.

9. Verwendung von Anspruch 8, wobei
(i) das mindestens eine Polynukleotid komplementär zu der mindestens einen miRNA von Anspruch 8 ist, oder
(ii) das mindestens eine Polynukleotid mindestens 80% Sequenzidentität mit dem Polynukleotid nach (i) hat.

10. Verwendung nach den Ansprüchen 8 oder 9, wobei die Blutzellfraktion Erythrozyten, Leukozyten und Thrombozyten umfasst.

11. Verwendung von mindestens einer miRNA, die aus einer Blutzellfraktion eines Patienten isoliert wurde, zur Diagnose von dreifach-negativem Brustkrebs (TNBC), wobei die mindestens eine miRNA aus der Gruppe bestehend aus SEQ ID NO: 1 bis SEQ ID NO: 23 und einer Sequenz mit mindestens 80% Sequenzidentität hierzu ausgewählt ist.

12. Verwendung nach Anspruch 11, wobei die Blutzellfraktion Erythrozyten, Leukozyten und Thrombozyten umfasst.

13. Verwendung eines Kits zur Diagnose von dreifach-negativem Brustkrebs (TNBC) in einer Blutzellfraktion eines Patienten, umfassend:
(i) Mittel zum Ermitteln des Levels von mindestens einer miRNA, die für den dreifach-negativen Brustkrebs repräsentativ ist, in einer Blutzellfraktion eines Patienten, und
(ii) optional ein Röhrchen zur Blutprobenlagerung,
wobei die mindestens eine miRNA aus der Gruppe bestehend aus SEQ ID NO: 1 bis SEQ ID NO: 23 und einer Sequenz mit mindestens 80% Sequenzidentität hierzu ausgewählt ist.

14. Verwendung des Kits nach Anspruch 13, wobei die Mittel zum Ermitteln des Levels der mindestens einen miRNA, die für den dreifach-negativen Brustkrebs repräsentativ ist, in einer Blutzellfraktion eines Patienten mindestens ein Polynukleotid, definiert gemäß einem der Ansprüche 8 bis 10, umfassen.

## Revendications

1. Méthode de diagnostic du cancer du sein triple négatif (TNBC) chez un patient comprenant l'étape de :
détermination du taux d'au moins un miARN représentatif du cancer du sein triple négatif dans une fraction cellulaire sanguine provenant du patient,
ledit au moins un miARN étant choisi dans le groupe constitué par SEQ ID NO : 1 à SEQ ID NO : 23, et une séquence présentant une identité de séquence d'au moins 80 % avec celles-ci.

2. Méthode selon la revendication 1, dans laquelle le taux dudit au moins un miARN est comparé à un taux de référence dudit au moins un miARN

3. Méthode selon la revendication 2, dans laquelle le taux de référence est le taux déterminé en mesurant au moins une fraction cellulaire sanguine de référence provenant d'au moins un sujet sain.

4. Méthode selon la revendication 3, dans laquelle :
le taux dudit au moins un miARN choisi dans le groupe constitué par SEQ ID NO : 1 à SEQ ID NO : 6, SEQ ID NO : 8 à SEQ ID NO : 14 et SEQ ID NO : 18 à SEQ ID NO : 23 au-dessus du taux de référence indique que le patient est atteint d'un cancer du sein triple négatif, et/ou
le taux dudit au moins un miARN choisi dans le groupe constitué par SEQ ID NO : 7 et SEQ ID NO : 15 à SEQ ID NO : 17 en dessous du taux de référence indique que le patient est atteint d'un cancer du sein triple négatif.

5. Méthode selon l'une quelconque des revendications 1 à 4, dans laquelle la fraction cellulaire sanguine comprend des érythrocytes, des leucocytes et des thrombocytes.

6. Méthode selon l'une quelconque des revendications 1 à 5, dans laquelle ledit au moins un miARN est choisi dans le groupe constitué par SEQ ID NO : 1 à SEQ ID NO : 8 et une séquence présentant une identité de séquence d'au moins 80 % avec celles-ci.

7. Méthode selon l'une quelconque des revendications 1 à 6, dans laquelle ledit au moins un miARN est un ou plusieurs ensembles de miARN, lesdits un ou plusieurs ensembles de miARN étant énumérés dans la figure 8.

8. Utilisation d'au moins un polynucléotide pour détecter au moins un miARN pour diagnostiquer le cancer du sein triple négatif (TNBC) dans une fraction cellulaire sanguine provenant d'un patient, dans laquelle ledit au moins un miARN est sélectionné dans le groupe constitué par SEQ ID NO : 1 à SEQ ID NO : 23.

9. Utilisation selon la revendication 8, dans laquelle
(i) ledit au moins un polynucléotide est complémentaire audit au moins un miARN selon la revendication 8, ou
(ii) ledit au moins un polynucléotide présente une identité de séquence d'au moins 80 % avec le polynucléotide selon (i).

10. Utilisation selon la revendication 8 ou 9, dans laquelle la fraction cellulaire sanguine comprend des érythrocytes, des leucocytes et des thrombocytes.

11. Utilisation d'au moins un miARN isolé à partir d'une fraction cellulaire sanguine provenant d'un patient pour diagnostiquer le cancer du sein triple négatif (TNBC),
ledit au moins un miARN étant choisi dans le groupe constitué par SEQ ID NO : 1 à SEQ ID NO : 23, et une séquence présentant une identité de séquence d'au moins 80 % avec celles-ci.

12. Utilisation selon la revendication 11, dans laquelle la fraction cellulaire sanguine comprend des érythrocytes, des leucocytes et des thrombocytes.

13. Utilisation d'un kit pour diagnostiquer le cancer du sein triple négatif (TNBC) dans une fraction cellulaire sanguine provenant d'une patient comprenant
(i) des moyens pour déterminer le taux d'au moins un miARN représentatif du cancer du sein triple négatif dans une fraction cellulaire sanguine provenant d'un patient, et
(ii) éventuellement un tube pour le stockage d'échantillons de sang,
ledit au moins un miARN étant choisi dans le groupe constitué par SEQ ID NO : 1 à SEQ ID NO : 23, et une séquence présentant une identité de séquence d'au moins 80 % avec celles-ci.

14. Utilisation du kit selon la revendication 13, dans laquelle les moyens pour déterminer le taux d'au moins un miARN représentatif du cancer du sein triple négatif dans une fraction cellulaire sanguine provenant d'un patient comprennent au moins un polynucléotide tel que défini dans l'une quelconque des revendications 8 à 10.
